(19) **Europäisches Patentamt / European Patent Office / Office européen des brevets**

(11) **EP 3 082 833 B1**

(12) **EUROPEAN PATENT SPECIFICATION**

(45) Date of publication and mention
of the grant of the patent:
**05.12.2018 Bulletin 2018/49**

(51) Int Cl.:
***G01N 33/92*** (2006.01)

(21) Application number: **14870759.9**

(86) International application number:
**PCT/US2014/071035**

(22) Date of filing: **18.12.2014**

(87) International publication number:
**WO 2015/095451 (25.06.2015 Gazette 2015/25)**

(54) **BIOMARKERS OF DE NOVO LIPOGENESIS AND METHODS USING THE SAME**

BIOMARKER FÜR DE-NOVO-LIPOGENESE UND VERFAHREN DAMIT

BIOMARQUEURS DE LIPOGÉNÈSE DE NOVO, ET LEURS PROCÉDÉS D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.12.2013 US 201361918866 P
31.01.2014 US 201461934033 P**

(43) Date of publication of application:
**26.10.2016 Bulletin 2016/43**

(73) Proprietor: **Metabolon, Inc.
Durham, NC 27713 (US)**

(72) Inventor: **WATKINS, Steven M.
Davis, CA 95618 (US)**

(74) Representative: **Murgitroyd & Company
Scotland House
165-169 Scotland Street
Glasgow G5 8PL (GB)**

(56) References cited:
WO-A2-2012/040318    US-A1- 2009 239 253
US-A1- 2009 239 253    US-A1- 2011 213 032
US-B2- 8 119 358

• KENNETH LYONS JONES ET AL: "Altered lipid metabolism in gastroschisis: A novel hypothesis", AMERICAN JOURNAL OF MEDICAL GENETICS PART A, vol. 161, no. 8, 21 June 2013 (2013-06-21), pages 1860-1865, XP055386023, US ISSN: 1552-4825, DOI: 10.1002/ajmg.a.36002
• WILLE J J ET AL: "PALMITOLEIC ACID ISOMER (C16:1DELTA6) IN HUMAN SKIN SEBUM IS EFFECTIVE AGAINST GRAM-POSITIVE BACTERIA", SKIN PHARMACOLOGY AND SKIN APPLIED PHYSIO, KARGER, BASEL, CH, vol. 16, 1 January 2003 (2003-01-01), pages 176-187, XP008052548, ISSN: 1422-2868, DOI: 10.1159/000069757
• RIME MICHAEL-JUBELI ET AL: "High-temperature gas chromatography-mass spectrometry for skin surface lipids profiling", JOURNAL OF LIPID RESEARCH, vol. 52, no. 1, 1 January 2011 (2011-01-01), pages 143-151, XP055386363, US ISSN: 0022-2275, DOI: 10.1194/jlr.D008094
• PETER, A ET AL.: 'Hepatic Glucokinase Expression Is Associated With Lipogenesis And Fatty Liver In Humans.' ENDOCRINOLOGY AND METABOLISM 13 April 2011, page E1127

EP 3 082 833 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field

Biomarkers, methods for identifying biomarkers correlated to de novo lipogenesis and methods based on the same biomarkers are described herein.

### Background

*De novo* lipogenesis (DNL) is the physiological process of synthesizing fatty acids from substrate, a process that in humans largely occurs in the liver. The process has been difficult to assess *in vivo*, because the amount of fat in blood is not in direct proportion to DNL. Lipogenesis contributes a minority of the fatty acids present in humans; most fatty acids come from diet, but the process of lipogenesis may be an important indicator of health status. A blood-based measure of lipogenesis would enable measurement of the impact of diet, lifestyle and therapies on DNL and assessment of the contribution of DNL to disease.

The absolute levels of individual fatty acids in blood are not indicative of DNL, because most fatty acids in humans are derived from diet, including fatty acids that are also the direct products of lipogenesis. Thus a direct measure of an individual fatty acid in blood cannot differentiate between the diet and DNL-derived origin of a fatty acid. In addition, the absolute level of all lipids in blood may be high or low depending on biology that is not related to DNL (for instance slow VLDL clearance).

To overcome this limitation, current procedures for measuring de novo lipogenesis involve the use of stable isotopes, particularly through the ingestion of heavy water. Hence, this method is not generally used in the clinic.

The diseases that involve DNL include diabetes and related conditions, obesity, hepatic steatosis, non-alcoholic steatohepatitis (NASH), cancer, cardiovascular disease (hypertriglyceridemia), and skin disorders. It is desirable to use minimally invasive or non-invasive methods to measure the level of fatty acids in blood and or skin that are indicative *of de novo* lipogenesis in humans, and normalize the effect of total blood lipid content in the assessment using these measured values to determine the amount of de novo lipogenesis of an individual.

Lyons Jones et al., Am J Med Genet, Part A, 161A:1860-1865 (2013) describes methods for assessing lipid metabolism in gastroschisis. Peter et al., J. Clin. Endocrinol. Metab. (2011), 96(7):E1126-E1130 investigates the relation between glucokinase expression and markers of lipogenesis and the use of a C16:0/C18:2 fatty acid DNL index. US2009/0239253 describes the use of various fatty acids in methods for assessing lipogenesis.

### Summary of the Invention

The present invention provides a method of estimating *de novo* lipogenesis as claimed in claim 1. Also described is a method of determining the level of de novo lipogenesis in a subject, the method comprising analyzing a biological sample from a subject to determine the level(s) of one or more biomarkers for de novo lipogenesis (DNL) in the sample, wherein the one or more biomarkers are selected from the group consisting of 16:1n10, 18:1n10, 18:1n12, squalene, 16:0, 16:1n7, 14:0, 14:1n5, 18:2n6, 20:4n6, and 22:6n3 and combinations thereof; and comparing the level(s) of the one or more biomarkers in the sample to DNL-positive and/or DNL-negative reference levels of the one or more biomarkers in order to determine the level of de novo lipogenesis in the subject.

Also described is a method of determining whether a subject is predisposed to or at risk of developing diseases that are related to de novo lipogenesis (e.g., diabetes, obesity, hepatic steatosis, non-alcoholic steatohepatitis (NASH), cancer, cardiovascular disease, hypertriglyceridemia) the method comprising analyzing a biological sample from a subject to determine the level(s) of one or more biomarkers for de novo lipogenesis (DNL) in the sample, wherein the one or more biomarkers are selected from the group consisting of 16:1n10, 18:1n10, 18:1n12, squalene, 16:0, 16:1n7, 14:0, 14:1n5, 18:2n6, 20:4n6, and 22:6n3 and combinations thereof; and comparing the level(s) of the one or more biomarkers in the sample to DNL-positive and/or DNL-negative reference levels of the one or more biomarkers in order to determine whether the subject is predisposed to developing said disease related to de novo lipogenesis.

Also described is a method of monitoring initiation / progression / regression of a DNL-related disease or disorder in a subject, the method comprising analyzing a biological sample from a subject to determine the level(s) of one or more biomarkers for DNL in the sample, where the one or more biomarkers are selected from the group consisting of 16:1n10, 18:1n10, 18:1n12, squalene, 16:0, 16:1n7, 14:0, 14:1n5, 18:2n6, 20:4n6, and 22:6n3 and combinations thereof; and comparing the level(s) of the one or more biomarkers in the sample to DNL progression and/or DNL regression reference levels in order to monitor the initiation/progression/regression of DNL-related disease or disorder in the subject.

Further described is a method of predicting whether a subject has a DNL-related disease or disorder comprising analyzing a biological sample from a subject to determine the level(s) of one or more DNL biomarkers in the sample, where the one or more biomarkers are selected from the group consisting of 16:1n10, 18:1n10, 18:1n12, squalene, 16:0, 16:1n7, 14:0, 14:1n5, 18:2n6, 20:4n6, and 22:6n3 and combinations thereof; and comparing the level(s) of the one or more biomarkers in the sample to DNL-positive and/or DNL-negative reference levels of the one or more biomarkers in order to predict whether the subject has a DNL-related disease or disorder.

The measurements of the one or more DNL biomarkers or combinations thereof may be used to generate a DNL Index that can be used to assess DNL in a subject. The method comprises, analyzing a biological sample from a subject to determine the level(s) of one or more biomarkers in the sample, wherein the one or more biomarkers are selected from the group consisting of 16:1n10, 18:1n10, 18:1n12, squalene, 16:0, 16:1n7, 14:0, 14:1n5, 18:2n6, 20:4n6, and 22:6n3 and combinations thereof, generating a mathematical model comprising the measured levels of said one or more biomarkers (DNL Index), calculating a DNL Index Score based on the DNL Index, and comparing the DNL Index Score to DNL reference levels in order to assess DNL in the subject.

Also described is a method of monitoring the efficacy of treatment for a DNL-related disease or disorder, the method comprising: analyzing a first biological sample from a subject to determine the level(s) of one or more DNL biomarkers in the sample, the first sample obtained from the subject at a first time point wherein the one or more biomarkers are selected from the group consisting of 16:1n10, 18:1n10, 18:1n12, squalene, 16:0, 16:1n7, 14:0, 14:1n5, 18:2n6, 20:4n6, and 22:6n3 and combinations thereof; treating the subject for a DNL-related disease or disorder; analyzing a second biological sample from the subject to determine the level(s) of the one or more biomarkers, the second sample obtained from the subject at a second time point after treatment; comparing the level(s) of one or more biomarkers in the first sample to the level(s) of the one or more biomarkers in the second sample to assess the efficacy of the treatment for treating a DNL-related disease or disorder.

Also described are methods of assessing lipogenesis in skin and its relationship to skin function (for example to assess sebum production, acne risk, etc), the method comprising analyzing a skin, epidermal, skin cell, or sebum sample from a subject to determine the level(s) of one or more DNL biomarkers in the sample, where the one or more biomarkers are selected from the group consisting of 16:1n10, 18:1n10, 18:1n12, squalene, 16:0, 16:1n7, 14:0, 14:1n5, 18:2n6, 20:4n6, and 22:6n3 and combinations thereof; and comparing the level(s) of the one or more biomarkers in the sample to DNL-positive and/or DNL-negative reference levels of the one or more biomarkers in order to assess the lipogenesis in the skin.

**Brief Description of the Drawings**

Figure 1 is an example medical algorithm for patient management illustrating where the DNL Index would be useful in clinical practice to determine aberrant levels of de novo lipogenesis, recommend treatment, monitor *de novo* lipogenesis and monitor the effect of treatment intervention on *de novo* lipogenesis.

Figure 2 is a graph showing the DNL Index Scores of cells treated with an inhibitor of DNL using the phospholipid fraction of cells. The box represents the middle 50% of the distribution, and upper and lower "whiskers" represent the entire spread of the data. The hyphen refers to the mean and the circle the outlier. The y-axis references the median scaled value.

Figure 3 is a boxplot graph showing the DNL Index Scores of cells treated with an inhibitor of DNL using the triglyceride fraction of cells.

Figure 4 is a boxplot graph showing the DNL Index Scores of cells treated with indicated doses of an inhibitor of DNL.

Figure 5 is a boxplot graph showing the DNL Index Scores of humans with NASH or NAFLD compared to normal control subjects.

Figure 6 shows the area under the curve (AUC) for three models to predict diabetes. Receiver operating characteristic curves show incremental ability of the DNL Index to predict diabetes in addition to acute insulin response (AIR), and insulin sensitivity (Si).

Figure 7 is a boxplot graph showing the sebum DNL Index Scores of skin treated with a placebo or an inhibitor of DNL using sebum DNL Index 1 and DNL Index 2.

Figure 8 is a graph showing the correlation of DNL Index Scores using DNL Index 1, DNL Index 2 and DNL Index 3 from human plasma samples with the fractional contribution of *de novo* lipogenesis (fDNL) in the patients.

Figure 9 is a boxplot display showing the distribution of values for the DNL Index Scores for NASH and Not-NASH patients. The NASH patients are on the left and the non-NASH patients are on the right. A. DNL Index 1, the p-value for the association with the DNL Index 1 Scores and the diagnosis of NASH was 0.027. B. DNL Index 2, the p-value for the association with the DNL Index 2 Scores and the diagnosis of NASH was 0.002. C. DNL Index 3, the p-value for the association with the DNL Index 3 Scores and the diagnosis of NASH was 0.004.

Figure 10 is a boxplot display showing the distribution of values for the DNL Index Scores for Steatosis Grade of patients. A. Is the plot of DNL Index 1; B. Is the plot of DNL Index 2; C. Is the plot of DNL Index 3. The DNL Index Score is on the y-axis and the degree of steatosis is on the x-axis. The p-value for the association with the DNL Index 1 Scores and the diagnosis of NASH was <0.001. The p-value for the association with the DNL Index 2 Scores and the diagnosis of NASH was <0.001. The p-value for the association with the DNL Index 3 Scores and the diagnosis of NASH was <0.001.

**Detailed Description of the Invention**

Biomarkers of DNL; methods for determining DNL; methods of diagnosing diseases related to DNL; methods of determining predisposition to diseases related to DNL; methods of monitoring progression/regression of diseases related to DNL; methods of predicting DNL related diseases or disorders; methods of generating a DNL Index; methods of monitoring the efficacy of treatments for DNL-related diseases; as well as other methods based on biomarkers of DNL are described herein.

Prior to describing this invention in further detail, however, the following terms will first be defined.

Definitions:

"De novo lipogenesis" or "DNL" refers to the physiological process of synthesizing fatty acids from substrate.

"Fractional DNL" or "fDNL" is a measurement of the fraction of newly synthesized fatty acids in VLDL-triacylglycerol. The method uses stable isotopes.

The "DNL Index" refers to a measure of de novo lipogenesis based upon the DNL biomarkers and medical algorithms described herein that will allow an assessment of de novo lipogenesis in vivo. "DNL Index" refers to a mathematical equation using the DNL biomarkers. "DNL Index Score" refers to the value obtained from or result of using a DNL Index.

"Medical Algorithm" or "Patient Management Algorithm" refers to any computation, formula, statistical survey, nomogram or look-up table useful in healthcare. Medical algorithms include decision tree approaches to healthcare treatment (e.g., if symptoms A, B, and C are evident, then use treatment X) and diagnosis (e.g., if symptoms E, F, and G are evident, then diagnosis is Z or diagnostic test to perform is Y). Medical algorihms may include diagnostic nomograms or diagnostic flowcharts in the form of, for example, a binary decision tree.

"DNL-related disease" or "DNL-related disorder" as used herein refers to diabetes and related conditions including pre-diabetes, insulin resistance, and type-2 diabetes, as well as obesity, hepatic steatosis, non-alcoholic steatohepatitis (NASH), cancer, and cardiovascular disease, including hypertriglyceridemia, atherosclerosis, cardiomyopathy, and skin disorders.

"Cardiovascular disease" refers to any disease of the heart or blood vessels. Cardiovascular or heart disease includes but is not limited to, for example, angina, arrhythmia, coronary artery disease (CAD), coronary heart disease, cardiomyopathy (including dilated cardiomyopathy, restrictive cardiomyopathy, arrhythmogenic right ventricular cardiomyopathy, and diabetic cardiomyopathy) heart attack (myocardial infarction), heart failure, hypertrophic cardiomyopathy, mitral regurgitation, mitral valve prolapse, pulmonary stenosis, etc. Blood vessel disease includes but is not limited to, for example, peripheral vascular disease, artery disease, carotid artery disease, deep vein thrombosis, venous diseases, atherosclerosis, etc.

"Diabetes" refers to a group of metabolic diseases characterized by high blood sugar (glucose) levels which result from defects in insulin secretion or action, or both.

"Type 2 diabetes" refers to one of the two major types of diabetes, the type in which the beta cells of the pancreas produce insulin, at least in the early stages of the disease, but the body is unable to use it effectively because the cells of the body are resistant to the action of insulin. In later stages of the disease the beta cells may stop producing insulin. Type 2 diabetes is also known as insulin-resistant diabetes, non-insulin dependent diabetes and adult-onset diabetes.

"Pre-diabetes" refers to one or more early diabetic conditions including impaired glucose utilization, abnormal or impaired fasting glucose levels, impaired glucose tolerance, impaired insulin sensitivity and insulin resistance.

"Insulin resistance" refers to the condition when cells become resistant to the effects of insulin - a hormone that regulates the uptake of glucose into cells - or when the amount of insulin produced is insufficient to maintain a normal glucose level. Cells are diminished in the ability to respond to the action of insulin in promoting the transport of the sugar glucose from blood into muscles and other tissues (i.e. sensitivity to insulin decreases). Eventually, the pancreas produces far more insulin than normal and the cells continue to be resistant. As long as enough insulin is produced to overcome this resistance, blood glucose levels remain normal. Once the pancreas is no longer able to keep up, blood glucose starts to rise, resulting in diabetes. Insulin resistance ranges from normal (insulin sensitive) to insulin resistant (IR).

"Insulin sensitivity" or "$S_i$" refers to the ability of cells to respond to the effects of insulin to regulate the uptake and utilization of glucose. Insulin sensitivity ranges from normal to Insulin Resistant (IR).

"Glucose utilization" refers to the absorption of glucose from the blood by muscle and fat cells and utilization of the sugar for cellular metabolism. The uptake of glucose into cells is stimulated by insulin.

"Obesity" refers to a chronic condition defined by an excess amount body fat. The normal amount of body fat (expressed as percentage of body weight) is between 25-30% in women and 18-23% in men. Women with over 30% body fat and men with over 25% body fat are considered obese.

"Skin disorders" refer to conditions affecting sebum production or quality including acne, seborrheic eczema and oily skin, or conditions affecting the stratum corneum including psoriasis, rosacea, dry skin, dandruff, water barrier function, etc. Skin disorders related to de novo lipogenesis may also include cancers such as melanomas.

"Body Mass Index, (BMI)" refers to a calculation that uses the height and weight of an individual to estimate the amount of the individual's body fat. Too much body fat (e.g. obesity) can lead to illnesses and other health problems. BMI is the measurement of choice for many physicians and researchers studying obesity. BMI is calculated using a mathematical formula that takes into account both height and weight of the individual. BMI equals a person's weight in kilograms divided by height in meters squared. ($BMI=kg/m^2$). Subjects having a BMI less than 19 are considered to be underweight, while those with a BMI of between 19 and 25 are considered to be of normal weight, while a BMI of between 25 and 29 is generally considered overweight, and individuals with a BMI of 30 or more are typically considered obese. Morbid obesity refers to a subject having a BMI of 40 or greater.

"Sample" or "biological sample" or "specimen" means biological material isolated from a subject. The biological sample may contain any biological material suitable for detecting the desired biomarkers, and may comprise cellular and/or non-cellular material from the subject. The sample can be isolated from any suitable biological tissue or fluid such as, for example, blood, blood plasma, serum, skin, epidermal tissue, adipose tissue, aortic tissue, liver tissue, urine, sebum, or cell samples.

"Subject" means any animal, but is preferably a mammal, such as, for example, a human, monkey, non-human primate, rat, mouse, cow, dog, cat, pig, horse, or rabbit.

The "level" of one or more biomarkers means the absolute or relative amount or concentration of the biomarker in the sample.

A "reference level" of a biomarker means a level of the biomarker that is indicative of a particular disease state, phenotype, or lack thereof, as well as combinations of disease states, phenotypes, or lack thereof. A "positive" reference level of a biomarker means a level that is indicative of a particular disease state or phenotype. A "negative" reference level of a biomarker means a level that is indicative of a lack of a particular disease state or phenotype. For example, a "DNL-positive reference level" of a biomarker means a level of a biomarker that is indicative of an increased measure of DNL in a subject, and a "DNL-negative reference level" of a biomarker means a level of a biomarker that is indicative of a decreased measure of DNL in a subject. As another example, a "DNL-progression-positive reference level" of a biomarker means a level of a biomarker that is indicative of progression of DNL or in a subject, and a "DNL-regression-positive reference level" of a biomarker means a level of a biomarker that is indicative of regression of DNL. A "reference level" of a biomarker may be an absolute or relative amount or concentration of the biomarker, a presence or absence of the biomarker, a range of amount or concentration of the biomarker, a minimum and/or maximum amount or concentration of the biomarker, a mean amount or concentration of the biomarker, and/or a median amount or concentration of the biomarker; and, in addition, "reference levels" of combinations of biomarkers may also be ratios of absolute or relative amounts or concentrations of two or more biomarkers with respect to each other. Appropriate positive and negative reference levels of biomarkers for a particular disease state, phenotype, or lack thereof may be determined by measuring levels of desired biomarkers in one or more appropriate subjects, and such reference levels may be tailored to specific populations of subjects (e.g., a reference level may be age-matched so that comparisons may be made between biomarker levels in samples from subjects of a certain age and reference levels for a particular disease state, phenotype, or lack thereof in a certain age group; similarly, a reference level may be matched to gender or ethnicity/race).

"DNL-positive marker" refers to a biomarker of de novo lipogenesis, the level of which is positively correlated with DNL (i.e., the level of the biomarker increases as the DNL increases).

"DNL-negative marker" refers to a biomarker of de novo lipogenesis, the level of which is negatively correlated with DNL (i.e., the level of the biomarker decreases as DNL increases).

## I. Biomarkers

Biomarkers for use in methods disclosed herein relating to DNL include the fatty acids 16:1n10, 18:1n10, 18:1n12, squalene, 16:0, 16:1n7, 14:0, 14:1n5, 18:2n6, 20:4n6, and 22:6n3 and combinations and subsets thereof. The biomarkers may be measured within a specific lipid class (e.g. triacylglycerides (TG), phospholipids (PL), cholesteryl esters (CE), diglycerides (DG), free fatty acids (FA), lysophosphatidylcholines (LY), phosphatidylcholines (PC), phosphatidylethanolamines (PE), sphingomyelins (SM), wax esters (WE), etc.) or as part of a total fatty acid analysis (including all lipid classes in the analysis). The fatty acids may be measured in any biological sample. 16:0, 16:1n7, 14:0, 14:1n5, 16:1n10, 18:1n10, 18:1n12 or intact complex lipids (e.g species of triacylglyceride or phospholipid) containing these fatty acids are positive markers of DNL (i.e., increased DNL is associated with elevated levels of the biomarkers), and 18:2n6, 20:4n6, 22:6n3 , intact complex lipids containing these fatty acids or squalene are negative markers of DNL (i.e., increased DNL is associated with reduced levels of the biomarkers). In one embodiment, the biomarkers include 16:0, 16:1n7, and 18:2n6, and combinations thereof.

In addition, the methods disclosed herein using the biomarkers 16:1n10, 18:1n10, 18:1n12, squalene, 16:0, 16:1n7, 14:0, 14:1n5, 18:2n6, 20:4n6, and 22:6n3 and combinations thereof may be used in combination with clinical diagnostic measures of the DNL-related diseases. Combinations with clinical diagnostics may facilitate the disclosed methods, or confirm results of the disclosed methods (for example, facilitating or confirming diagnosis, monitoring progression or regression, and/or determining predisposition to DNL-related diseases). The methods disclosed herein using the biomarkers 16:1n10, 18:1n10, 18:1n12, squalene, 16:0, 16:1n7, 14:0, 14:1n5, 18:2n6, 20:4n6, and 22:6n3 and combinations thereof may also be used in combination with patient information such as, for example gender, race, age, medical history, family medical history, risk factors, etc.

Methods of measuring the fatty acids disclosed herein as components of a class of intact complex lipids (e.g. species of triacylglycerides or phospholipids such as PC16:0|16:1n7) are also contemplated. The lipid class may be, for example, neutral lipids, phospholipids, free fatty acids, total fatty acids, triglycerides, cholesteryl esters, phosphatidylcholines, phosphatidylethanolamines, diglycerides, lysophosphatidylcholines, or wax esters. In some embodiments, the lipid class is selected from the group consisting of neutral lipids, phospholipids, free fatty acids, total fatty acids, triglycerides, cholesterol esters, phosphatidylcholines, and phosphatidylethanolamines. In some embodiments, the lipid class is selected from the group consisting of neutral lipids, phospholipids, total fatty acids, and cholesterol esters. In some embodiments, the lipid class is selected from the group consisting of free fatty acids, total fatty acids, triglycerides, cholesterol esters, phosphatidylcholines, and phosphatidylethanolamines. In some embodiments, the lipid class is selected from the group consisting of triglycerides, free fatty acids, and wax esters. In some embodiments, the lipid class is free fatty acids. In some embodiments, the lipid class is total fatty acids. In some embodiments, the lipid class is triglycerides. In some embodiments, the lipid class is cholesteryl esters. In some embodiments, the lipid class is phosphatidylcholines. In some embodiments, the lipid class is phosphatidylethanolamines. In some embodiments, the lipid class is phospholipids. In some embodiments, the lipid class is neutral lipids. In some embodiments, the lipid class is diglycerides. In some embodiments, the lipid class is sphingomyelins. In some embodiments, the lipid class is wax esters. The prefixes "TG", "FA", "PC", "PE", and "CE" correspond to fatty acids present within triglycerides, free fatty acids, phosphatidylcholines, phosphatidylethanolamines, and cholesterol esters, respectively. Thus, "TG14:0" indicates the fatty acid 14:0 present within triglycerides.

## II. Assessing DNL

The DNL biomarkers can be used to assess (or aid in the assessment of) DNL in a subject. It will be understood that the identified biomarkers can be used to assess DNL in any subject and includes the assessment of DNL in a healthy subject (for example, as part of a routine physical health assessment), in an asymptomatic subject, in a subject suspected of having or at risk for a DNL-related disease, or in a subject in response to a composition or to a therapeutic intervention. It is further understood that a subject may undergo one or more assessments of DNL.

In an exemplary method, assessing DNL in a subject comprises (1) analyzing a biological sample obtained from a subject to determine the level(s) of one or more biomarkers for DNL in the sample and (2) comparing the level(s) of the one or more biomarkers in the sample to reference level(s) of the one or more biomarkers to assess the level of DNL in a subject. The one or more biomarkers may be selected from the group consisting of 16:1n10, 18:1n10, l8:1n12, squalene, 16:0, 16:1n7, 14:0, 14:1n5, 18:2n6, 20:4n6, and 22:6n3 and combinations thereof. For example, the level(s) of one biomarker, two or more biomarkers, three or more biomarkers, four or more biomarkers, five or more biomarkers, six or more biomarkers, etc., including a combination of all of the listed biomarkers may be used in assessing DNL. Determining levels of combinations of the biomarkers may allow greater sensitivity and specificity in the methods described herein. For example, pair-wise analysis of two biomarkers or ratios of the levels of certain biomarkers (and non-biomarker compounds) in biological samples may allow greater sensitivity and specificity in assessing DNL.

Further, the present disclosure describes methods of assessing DNL in skin and its relationship to skin function (for example to assess sebum production, acne risk, etc). In an exmplary method, assessing DNL in a subject comprises analyzing a skin or epidermal or skin cell sample from asaid subject to determine the level(s) of one or more DNL biomarkers in the sample, where the one or more biomarkers are selected from the group consisting of 16:1n10, 18:1n10, 18:1n12, squalene, 16:0, 16:1n7, 14:0, 14:1n5, 18:2n6, 20:4n6, and 22:6n3 and combinations thereof; and comparing the level(s) of the one or more biomarkers in the sample to DNL-positive and/or DNL-negative reference levels of the one or more biomarkers in order to assess the lipogenesis in the skin. For example, the level(s) of one biomarker, two or more biomarkers, three or more biomarkers, four or more biomarkers, five or more biomarkers, six or more biomarkers, etc., including a combination of all of the listed biomarkers may be used in assessing DNL. In one embodiment the one or more biomarkers are selected from the group consisting of 16:1n10, 18:1n10, 18:1n12, and squalene. In another embodiment the one or more biomarkers selected from the goup consisting of 16:1n10, 18:1n10, 18:1n12, and squalene are used in combinations with one or more biomarkers selected from the group consisting of 16:0, 16:1n7, 14:0, 14:1n5, 18:2n6, 20:4n6, and 22:6n3 and combinations thereof. In another embodiment, the one or more biomarkers comprise 16:0, 16:1n7, and 18:2n6. In another embodiment, the one or more biomarkers comprise 16:0, 16:1n10, and 18:2n6. In another embodiment, the one or more biomarkers comprise 16:0, 16:1n10, and squalene. In another embodiment, the one or more biomarkers comprise 16:0, 16:1n7, 18:2n6, 14:0, and 14:1n5. In another embodiment, the one or more biomarkers comprise 16:0, 16:1n7, 18:2n6, 14:0, 14:1n5, 20:4n6, and 22:6n3. Determining levels of combinations of the biomarkers may allow greater sensitivity and specificity in the methods described herein. For example, pair-wise analysis of two biomarkers or ratios of the levels of certain biomarkers (and non-biomarker compounds) in biological samples may allow greater sensitivity and specificity in assessing DNL.

Any suitable method may be used to analyze the biological sample in order to determine the level(s) of the one or more biomarkers in the sample. Suitable methods include chromatography (e.g., HPLC, gas chromatography, capillary gas chromatography, liquid chromatography), mass spectrometry (e.g., MS, MS-MS), enzyme-linked immunosorbent assay (ELISA), antibody linkage, other immunochemical techniques, and combinations thereof.

The level(s) of the one or more biomarkers may be compared to DNL reference levels using various techniques, including a simple comparison (e.g., a manual comparison). The level(s) of the one or more biomarkers in the biological sample may also be compared to reference levels using one or more statistical analyses (e.g., t-test, Welch's T-test, Wilcoxon's rank sum test, correlation analysis, Random Forest, T-score, Z-score) or using a mathematical model (e.g., statistical model). For example, a mathematical model comprising a single biomarker analyte measurement or multiple biomarker analyte measurements may be used to assess DNL in a subject. When analyzing the effects rendered by two or more DNL-biomarkers, one can either evaluate the effects of these biomarkers individually or obtain the net effect of these biomarkers, e.g., by using various mathematical formulas or models to quantify the effect of each biomarker. A formula containing the levels of one or more DNL-biomarkers as variables includes any mathematical formula, model, equation, or expression established based on mathematic or statistical principles or methods using the values of one or more biomarker as variables.

The results of the method may be used along with other methods (or the results thereof) useful in the assessment of DNL in a subject. For example, fDNL, insulin sensitivity and/or acute insulin response (AIR) measurements as well as patient information such as, for example, age, BMI, gender, race or other risk factors can be used with the biomarkers.

The biomarkers provided herein can be used in a mathematical or statistical model or formula ("DNL Index") to generate a numerical score ("DNL Index Score") which is an indicator of DNL in the subject. The DNL Index Score places the subject in a range of DNL from low to normal to high. Methods for generating a DNL Index may comprise obtaining biological samples from one or more reference cohorts (e.g., healthy individuals, individuals with DNL-realted diseases), measuring the levels of one or more DNL biomarkers in the samples and using the measured levels in a mathematical model comprised of the said measured levels of the one or more biomarkers. For example, methods such as multivariate analysis of variance, multivariate regression, multiple regression can be used to determine relationships between dependent variables, and independent variables in the DNL Index. The method may employ any number of markers selected from 16:1n10, 18:1n10, 18:1n12, squalene, 16:0, 16:1n7, 14:0, 14:1n5, 18:2n6, 20:4n6, and 22:6n3 and combinations thereof. Multiple biomarkers may be correlated with DNL, by any method, including statistical methods such as regression analysis.

In some embodiments, a formula containing one or more DNL-biomarkers as variables is established by using regression analyses, e.g., multiple linear regressions. In one embodiment, the biomarkers may be used in a statistical model to generate a DNL Index. For example, without any limitation, the following equations may be used in a DNL Index to generate a DNL Index Score:

$$\text{DNL Index Score} = a(16{:}0) + b(16{:}1n7) - c(18{:}2n6) \text{ (using mole\%}$$
$$\text{data or normalizing for total or TG)}$$

$$\text{DNL Index Score} = (a(16{:}0) + b(16{:}1n7))/c(18{:}2n6) \text{ (normalizing}$$
$$\text{ratio)}$$

$$\text{DNL Index Score} = a(\text{DNL positive marker}) - b(\text{DNL negative}$$
$$\text{marker})$$

$$\text{DNL Index Score} = a(16{:}0) + b(16{:}1n10) - c(18{:}2n6)$$

$$\text{DNL Index Score} = a(16{:}0) + b(16{:}1n10) - c(\text{squalene})$$

$$\text{DNL Index Score} = a(16{:}0) + b(16{:}1n7) - c(18{:}2n6)$$

$$\text{DNL Index Score} = a(16{:}0) + b(16{:}1n7) - c(18{:}2n6) + d(14{:}0) + e(14{:}1n5)$$

$$\text{DNL Index Score} = a(16{:}0) + b(16{:}1n7) - c(18{:}2n6) + d(14{:}0) + e(14{:}1n5) - f(20{:}4n6) - g(22{:}6n3)$$

Where a, b, c, d, e, f, g are coefficients that serve as scaling factors that can be estimated from standard human levels of each of the markers. Further, the result of the equation (DNL Index Score) can be scaled (e.g. from 1-10) to provide optimal clinical utility. The biomarker metabolites can be expressed in quantitative or relative terms (e.g., mole%); however, the coefficients and the form of the equation must accommodate the form of the data provided. The formulas may use one or more DNL-biomarkers as variables, such as 1, 2, 3, 4, 5 or more biomarkers. The constants of these formulas can be established by using a set of data obtained from known DNL values or from cohorts having DNL-associated diseases. The levels of DNL-biomarkers used in these formulas can be either the levels at a time point or changes of levels over a period of time.

In another aspect, the DNL Index may incorporate variables such as, for example, gender and/or race. In another aspect, the DNL Index may incorporate the measurements for additional fatty acids or lipids.

The DNL Index Score can be used to classify the subject according to level of DNL (e.g., normal, low, high). Non-limiting example uses of the DNL Index Score include: assessment of DNL; classification of DNL; predisposition to developing DNL-related diseases; diagnosis of DNL-related diseases; monitoring progression/regression of DNL-related diseases; and monitoring the efficacy of treatment for DNL-related diseases.

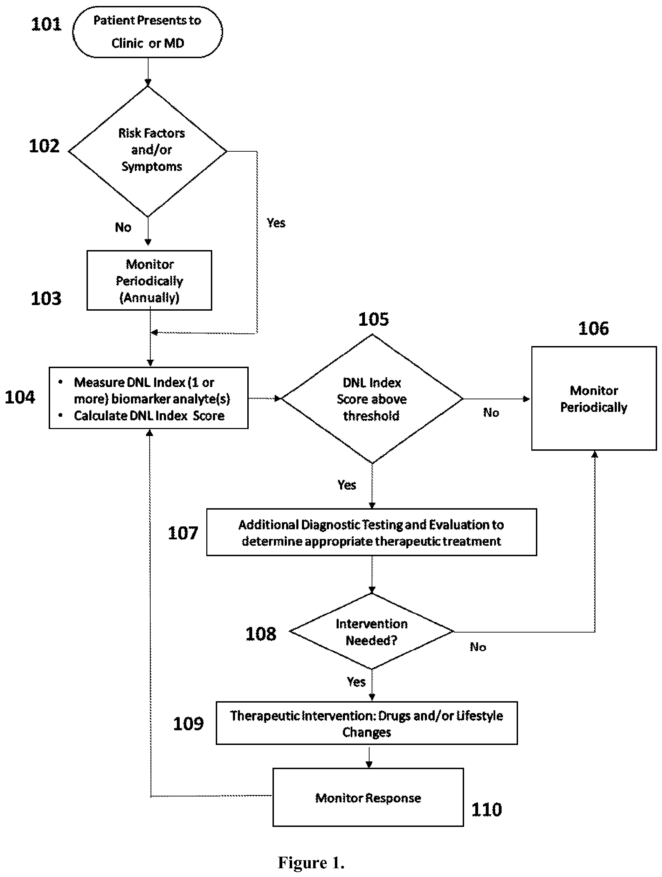

In a further aspect, the DNL biomarkers or DNL Index may be included in a medical algorithm for patient management where the DNL Index and Score would be a useful metric to integrate into clinical practice. The medical algorithm may use the measured value of one or more DNL biomarker analytes or the calculated DNL Index Score to guide the additional testing, evaluation and treatment of a subject presenting to a clinic or physician. A non-limiting and simple example algorithm is presented in Figure 1. The subject (101) may present to the clinic with symptoms or may be asymptomatic (102). The subject may have risk factors such as a personal history or a family history of aberrant de novo lipogenesis or may not have risk factors (102). If the subject has no risk factors and is asymptomatic, then the DNL Index will not be determined and the patient will be monitored annually (103) unless symptoms or risk factors develop. If the subject is presently symptomatic and/or has risk factors (e.g., a personal or family history), then a sample from the subject will be analyzed (104) to determine said subjects's DNL Index Score (105). Similarly, if the subject is asymptomatic and has risk factors (e.g., a personal or family history of the same), then a sample from the subject will be analyzed (104) to determine said subjects's DNL Index Score (105). If the DNL Index Score (105) is not above a threshold value or reference value indicating the presence of DNL or predisposition to DNL-related disease, then the subject will be monitored periodically (106). If the DNL Index Score (105) is above a threshold value or reference value indicating the presence of DNL or predisposition to DNL-related disease, then the subject will be referred for additional testing and evaluation (107) to determine if said subject should be treated (e.g., with drugs and/or lifestyle changes). If it is determined based on the additional testing and evaluation that treatment is needed (108), then therapeutic intervention will be prescribed (109) and said patient's response to therapy may then be monitored (110). Said subject will return periodically for determination of their DNL status by measuring DNL biomarkers (104) and calculating a DNL Score (105). The DNL Score will be compared to the prior DNL Score to determine if the treatment is effective. If it is determined that treatment intervention (108) is not required, said subject will be monitored periodically (106).

### III. Monitoring Disease Progression / Regression

The use of DNL biomarkers in a DNL Index allows for monitoring progression/regression of DNL-related diseases (e.g. diabetes and related conditions, obesity, hepatic steatosis, non-alcoholic steatohepatitis (NASH), cancer, and cardiovascular disease, etc.) in a subject. A method of monitoring the progression/regression of a DNL-related disease, such as diabetes and related conditions, obesity, hepatic steatosis, non-alcoholic steatohepatitis (NASH), cancer, cardiovascular disease, and skin disorders in a subject comprises (1) analyzing a first biological sample from a subject to determine the level(s) of one or more biomarkers for DNL selected from the group consisting of 16:1n10, 18:1n10, 18:1n12, squalene,16:0, 16:1n7, 14:0, 14:1n5, 18:2n6, 20:4n6, and 22:6n3 and combinations thereof in the first sample obtained from the subject at a first time point, (2) analyzing a second biological sample from a subject to determine the level(s) of the one or more biomarkers, the second sample obtained from the subject at a second time point, and (3) comparing the level(s) of one or more biomarkers in the second sample to (a) the level(s) of the one or more biomarkers in the first sample and/or (b) to DNL reference levels in order to monitor the progression/regression of the DNL-related disease in the subject. The results of the method are indicative of the course of the DNL-related disease (i.e., progression or regression, if any change) in the subject.

After the first sample is obtained one or more additional samples may be obtained from the subject at a later point in time. In one aspect, the one or more additional samples are obtained 1, 2, 3, 4, 5, 6, or more days after the first sample. In another aspect, the one or more samples is obtained 1, 2, 3, 4, 5, 6, 7, 8, 9, 10, or more weeks after the first sample or after the initiation of treatment with the composition. In another aspect, the one or more additional samples may be obtained 1, 2, 3, 4, 5, 6, 7, 8, 9, 10, 11, 12, or more months after the first sample or after the initiation of treatment with the composition.

In one embodiment, the results of the method may be based on the DNL Index Score which is indicative of the level of DNL in the subject and which can be monitored over time. By comparing the DNL Index Score from a first time point sample to the DNL Index Score from at least a second time point sample the progression or regression of DNL-related disease can be determined. For example, such a method of monitoring the progression/regression of pre-diabetes and/or type-2 diabetes in a subject comprises (1) analyzing a first biological sample from a subject to determine a DNL Index Score for the first sample obtained from the subject at a first time point, (2) analyzing a second biological sample from a subject to determine a second DNL Index Score, the second sample obtained from the subject at a second time point, and (3) comparing the DNL Index Score in the first sample to the DNL Index Score in the second sample in order to monitor the progression/regression of pre-diabetes and/or type-2 diabetes in the subject.

Using the biomarkers and DNL Index described herein for progression monitoring may guide, or assist a physician's decision to implement preventative measures such as dietary restrictions, exercise, or early-stage drug treatment.

## IV. Determining Predisposition to Disease

The use of DNL biomarkers in a DNL Index as described herein may also be used in the determination of whether a subject not exhibiting any symptoms of a disease related to DNL, such as diabetes and related conditions, obesity, hepatic steatosis, non-alcoholic steatohepatitis (NASH), cancer, cardiovascular disease, and skin disorders, is predisposed to developing a DNL-related disease. Such methods of determining whether a subject having no symptoms of a DNL-related disease such as diabetes and related conditions, obesity, hepatic steatosis, non-alcoholic steatohepatitis (NASH), cancer, cardiovascular disease, and skin disorders, is predisposed to developing a DNL-related disease comprise (1) analyzing a biological sample from a subject to determine the level(s) of one or more biomarkers selected from the group consisting of 16:1n10, 18:1n10, 18:1n12, squalene, 16:0, 16:1n7, 14:0, 14:1n5, 18:2n6, 20:4n6, and 22:6n3 and combinations thereof in the sample and (2) comparing the level(s) of the one or more biomarkers in the sample to DNL-positive and/or DNL-negative reference levels of the one or more biomarkers in order to determine whether the subject is predisposed to developing a DNL-related disease. The biomarkers may also be used in a mathematical or statistical model or formula (DNL Index) to determine predisposition to a DNL-related disease. The results of the method may be used along with other methods (or the results thereof) useful in the clinical determination of whether a subject is predisposed to developing the disease.

After the level(s) of the one or more biomarkers in the sample are determined, the level(s) are compared to DNL-positive and/or DNL-negative reference levels in order to predict whether the subject is predisposed to developing a DNL-related disease such as diabetes and related conditions, obesity, hepatic steatosis, non-alcoholic steatohepatitis (NASH), cancer, cardiovascular disease, or skin disorders. Levels of the one or more biomarkers in a sample corresponding to DNL-positive reference levels (e.g., levels that are the same as the reference levels, substantially the same as the reference levels, above and/or below the minimum and/or maximum of the reference levels, and/or within the range of the reference levels) are indicative of the subject being predisposed to developing the DNL-related disease. Levels of the one or more biomarkers in a sample corresponding to DNL-negative reference levels (e.g., levels that are the same as the reference levels, substantially the same as the reference levels, above and/or below the minimum and/or maximum of the reference levels, and/or within the range of the reference levels) are indicative of the subject not being predisposed to developing the DNL-related disease. In addition, levels of the one or more biomarkers that are differentially present (especially at a level that is statistically significant) in the sample as compared to DNL-negative reference levels may be indicative of the subject being predisposed to developing the DNL-related disease. Levels of the one or more biomarkers that are differentially present (especially at a level that is statistically significant) in the sample as compared to DNL-positive reference levels are indicative of the subject not being predisposed to developing the DNL-related disease.

In another embodiment, the biomarkers may be used in a mathematical or statistical model or formula (DNL Index) to determine predisposition to a DNL-related disease, the method comprising (1) analyzing a biological sample from a subject to determine the level(s) of one or more biomarkers selected from the group consisting of 16:1n10, 18:1n10, 18:1n12, squalene, 16:0, 16:1n7, 14:0, 14:1n5, 18:2n6, 20:4n6, and 22:6n3 and combinations thereof in the sample, (2) using a mathematical model comprising the measured levels of said one or more biomarkers to generate a DNL Index, (3) calculating a DNL Index Score from said DNL Index, and (4) comparing the DNL Index Score from the sample to DNL-positive and/or DNL-negative reference levels of the one or more biomarkers in order to determine whether the subject is predisposed to developing a DNL-related disease.

Furthermore, it may also be possible to determine reference levels specific to assessing whether or not a subject that does not have a DNL-related disease such as diabetes and related conditions, obesity, hepatic steatosis, non-alcoholic steatohepatitis (NASH), cancer, cardiovascular disease, or skin disorders is predisposed to developing a DNL-related disease. For example, it may be possible to determine reference levels of the biomarkers for assessing different degrees of risk (e.g., low, medium, high) in a subject for developing a DNL-related disease. Such reference levels could be used for comparison to the levels of the one or more biomarkers in a biological sample from a subject.

### V. Monitoring Therapeutic Efficacy

The biomarkers provided also allow for the assessment of the efficacy of treatment for a DNL-related disease such as diabetes and related conditions, obesity, hepatic steatosis, non-alcoholic steatohepatitis (NASH), cancer, cardiovascular disease, or skin disorders. Such assessments may be used, for example, in efficacy studies, in monitoring the effects of lifestyle modifications, as well as in lead selection of compositions for treating the DNL-related disease. For example, the use of DNL biomarkers in a DNL Index for diabetes also allows for assessment of the efficacy of a treatment for diabetes as well as the assessment of the relative efficacy of two or treatments for diabetes. Treatments to be monitored for therapeutic efficacy may include diet, lifestyle modifications, treatment with a composition, or other therapies used to treat DNL-related conditions.

Thus, also described are methods of assessing the efficacy of a treatment for a DNL-related disease such as diabetes and related conditions, obesity, hepatic steatosis, non-alcoholic steatohepatitis (NASH), cancer, cardiovascular disease, or skin disorders comprising (1) analyzing, from a subject (or group of subjects) having a DNL-related disease such as diabetes and related conditions, obesity, hepatic steatosis, non-alcoholic steatohepatitis (NASH), cancer, cardiovascular disease, or skin disorders and currently or previously undergoing treatment for said disease, a biological sample (or group of samples) to determine the level(s) of one or more biomarkers for the disorder selected from the group consisting of 16:1n10, 18:1n10, 18:1n12, squalene, 16:0, 16:1n7, 14:0, 14:1n5, 18:2n6, 20:4n6, and 22:6n3 and combinations thereof, and (2) comparing the level(s) of the one or more biomarkers in the sample to (a) level(s) of the one or more biomarkers in a previously-taken biological sample from the subject, wherein the previously-taken biological sample was obtained from the subject before starting the treatment, (b) DNL-positive reference levels of the one or more biomarkers, (c) DNL-negative reference levels of the one or more biomarkers, (d) DNL-progression-positive reference levels of the one or more biomarkers, and/or (e) DNL-regression-positive reference levels of the one or more biomarkers. The results of the comparison are indicative of the efficacy of the treatment for the DNL-related disease.

The second sample may be obtained from the subject any period of time after the first sample is obtained. In one aspect, the second sample is obtained 1, 2, 3, 4, 5, 6, or more days after the first sample or after the initiation the therapy. In another aspect, the second sample is obtained 1, 2, 3, 4, 5, 6, 7, 8, 9, 10, or more weeks after the first sample or after the initiation of the therapy. In another aspect, the second sample may be obtained 1, 2, 3, 4, 5, 6, 7, 8, 9, 10, 11, 12, or more months after the first sample or after the initiation of the therapy.

The change (if any) in the level(s) of the one or more biomarkers over time may be indicative of progression or regression of the DNL-related disease in the subject. To characterize the course of a given DNL-related disease in the subject, the level(s) of the one or more biomarkers in the first sample, the level(s) of the one or more biomarkers in the second sample, and/or the results of the comparison of the levels of the biomarkers in the first and second samples may be compared to DNL-positive and/or DNL-negative reference levels of the one or more biomarkers. If the comparisons indicate that the level(s) of the one or more biomarkers are increasing or decreasing over time (e.g., in the second sample as compared to the first sample) to become more similar to the DNL-positive reference levels (or less similar to the DNL-negative reference levels), then the results are indicative of progression of the DNL-related disease. If the comparisons indicate that the level(s) of the one or more biomarkers are increasing or decreasing over time to become more similar to the DNL-negative reference levels (or less similar to the DNL-positive reference levels), then the results are indicative of regression of the DNL-related disease.

In another embodiment, the biomarkers may be used in a mathematical or statistical model or formula (DNL Index) to determine the efficacy of a treatment for a DNL-related disease, the method comprising, (1) analyzing, from a subject (or group of subjects) having a DNL-related disease such as diabetes and related conditions, obesity, hepatic steatosis, non-alcoholic steatohepatitis (NASH), cancer, cardiovascular disease, or skin disorders and currently or previously undergoing treatment for said disease, a biological sample (or group of samples) to determine the level(s) of one or more biomarkers for the disorder selected from the group consisting of 16:1n10, 18:1n10, 18:1n12, squalene, 16:0, 16:1n7, 14:0, 14:1n5, 18:2n6, 20:4n6, and 22:6n3 and combinations thereof, (2) using a mathematical model comprising the measured levels of said one or more biomarkers to generate a DNL Index, (3) calculating a DNL Index Score from said DNL Index, and and (4) comparing the level(s) of the one or more biomarkers in the sample to (a) level(s) of the one or more biomarkers in a previously-taken biological sample from the subject, wherein the previously-taken biological sample was obtained from the subject before starting the treatment, (b) DNL-positive reference levels of the one or more biomarkers, (c) DNL-negative reference levels of the one or more biomarkers, (d) DNL-progression-positive reference levels of the one or more biomarkers, and/or (e) DNL-regression-positive reference levels of the one or more biomarkers. The results of the comparison are indicative of the efficacy of the treatment for the DNL-related disease.

As with the other methods described herein, the comparisons made in the methods of monitoring therapeutic efficacy of a treatment for a DNL-related disease such as diabetes and related conditions, obesity, hepatic steatosis, non-alcoholic steatohepatitis (NASH), cancer, cardiovascular disease, or skin disorders in a subject may be carried out using various techniques, including simple comparisons, one or more statistical analyses, and combinations thereof.

The results of the method may be used along with other methods (or the results thereof) described herein and/or other methods useful in the clinical monitoring of progression/regression of the disease or condition in a subject.

As described above in connection with methods of diagnosing (or aiding in the diagnosis of) a disease or condition such as diabetes and related conditions, obesity, hepatic steatosis, non-alcoholic steatohepatitis (NASH), cancer, cardiovascular disease, or skin disorders, any suitable method may be used to analyze the biological samples in order to determine the level(s) of the one or more biomarkers in the samples. In addition, the level(s) one or more biomarkers, including a combination of all of the biomarkers selected from the group consisting of 16:1n10, 18:1n10, 18:1n12, squalene, 16:0, 16:1n7, 14:0, 14:1n5, 18:2n6, 20:4n6, and 22:6n3, or any fraction thereof, may be determined and used in methods of monitoring progression/regression of the DNL-related disease in a subject.

Such methods could be conducted to monitor the course of disease or condition development in subjects, for example the course of pre-diabetes to type-2 diabetes in a subject having pre-diabetes, or could be used in subjects not having a disease or condition (e.g., subjects suspected of being predisposed to developing the disease or condition) in order to monitor levels of predisposition to the disease or condition.

The biomarkers provided also allow for the identification of subjects in whom the treatment for a DNL-related disease such as diabetes and related conditions, obesity, hepatic steatosis, non-alcoholic steatohepatitis (NASH), cancer, cardiovascular disease, or skin disorders is efficacious (i.e. patient responds to therapy). Such assessments may be used, for example, in selection of compositions for treating the disease or condition for certain subjects.

## VI. Methods of Using the Biomarkers for Other Diseases or Conditions

In another aspect, at least some of the biomarkers disclosed herein for DNL may also be biomarkers for other diseases or conditions not currently known to be associated with DNL. That is, the methods described herein with respect to DNL may also be used for diagnosing (or aiding in the diagnosis of) a disease or condition suspected of being related to DNL, methods of monitoring progression/regression of such a disease or condition, methods of determining predisposition of such a disease or condition, and methods of assessing efficacy of compositions for treating such a disease or condition. Such methods could be conducted as described herein with respect to insulin resistance.

## Examples

## I. General Methods

Briefly, the lipids from plasma samples or sebum samples were extracted in the presence of authentic internal standards by the method of Folch et al. [Folch, J., M. Lees, and G. H. Sloane-Stanley. 1957. A simple method for the isolation and purification of total lipids from animal tissues. J. Biol. Chem. 226: 497-509.] using chloroform:methanol (2:1 v/v). The sebum samples were collected using sebutape as described [Pierard, G.E., Pierard-Franchimont, C. & Kligman, A.M. Kinetics of sebum excretion evaluated by the Sebutape--Chromameter technique. Skin pharmacology : the official journal of the Skin Pharmacology Society 6, 38-44 (1993)] The total lipid extract was trans-esterified in 1% sulfuric acid in methanol in a sealed vial under a nitrogen atmosphere at 100°C for 45 min. The resulting extract was neutralized with 6% potassium carbonate and the fatty acid methyl esters (FAME) were extracted with hexane and prepared for gas chromatography. Fatty acid methyl esters were separated and quantified by capillary gas chromatography (Agilent Technologies model 6890) equipped with a 30 m DB-88MS capillary column (Agilent Technologies) and a flame-ionization detector. Quantitative results were obtained by comparing each fatty acid to its internal standard control. All assays presented here passed internal quality control protocols.

Mole percent data (mole%) was used for all analyses in these Examples. Mole% data are simply fatty acid composition data, with each fatty acid expressed as a percentage of the total pool. By converting quantitative fatty acid concentrations to mole% concentrations, the effect of changes in total plasma lipid levels (e.g. increased or decreased triacylglycerides) was normalized away. Alternatively, these fatty acids may be measured as components of intact complex lipids (e.g. species of triacylglycerides or phospholipids such as PC16:0|16:1n7).

**Example 1: De Novo Lipogenesis (DNL) Index**

Several fatty acids are affected by *de novo* lipogenesis, some in a positive way (i.e., they are synthesized *de novo*), and some in a negative way (i.e., they are diluted by *de novo* lipogenesis or are inhibitors of the process). For example, the fatty acids palmitate and palmitoleate (16:0 and 16:1n7) are products of human DNL (Aarsland A, Wolfe RR. Hepatic secretion of VLDL fatty acids during stimulated lipogenesis in men. J Lipid Res. 1998;39:1280-1286.; Wu JH, Lemaitre RN, Imamura F, King IB, Song X, Spiegelman D, Siscovick DS, Mozaffarian D. Fatty acids in the de novo lipogenesis pathway and risk of coronary heart disease: the Cardiovascular Health Study. Am J Clin Nutr. 2011;94:431-438.). Myristate and myristoleate are the 14 carbon analogues of 16:0 and 16:1n7 and are also produced via *de novo* lipogenesis except in much lower abundance. However, linoleic acid (18:2n6) is exclusively derived from diet (it is the most abundant polyunsaturated fatty acid in the food supply). 18:2n6 was strongly but negatively correlated with other members of the set, suggesting that the cluster represents the balance of DNL-derived and dietary derived fatty acids in circulation. Previous studies have shown decreased levels of 18:2n6 in lipids of DNL origin (Aarsland A, Wolfe RR. Hepatic secretion of VLDL fatty acids during stimulated lipogenesis in men. J Lipid Res. 1998;39:1280-1286.).

Fatty acids related to DNL include: (A) Positive markers: 16:0, 16:1n7, 14:0, 14:1n5 and (B) Negative markers: 18:2n6, 20:4n6, 22:6n3.

The most consistent biomarkers of DNL were: 16:0, 16:1n7 and 18:2n6. These markers were selected as exemplary biomarkers to measure the levels in samples and for use of said measured levels in an Index to measure DNL. The biomarkers can be measured within a specific lipid class (e.g. triacylglycerides (TG), phospholipids (PL), cholesteryl esters (CE)) or as part of a total fatty acid analysis (including all lipid classes in the analysis). The fatty acids can be measured in tissues or fluids, and are particularly useful biomarkers of DNL when measured from serum or plasma. In this example, the measured values of these biomarkers are used in statistical model to generate a DNL Index which is useful to inform the clinician about the DNL status of the patient, monitor response to therapeutic intervention, assess predisposition to diseases such as diabetes and related conditions, obesity, hepatic steatosis, non-alcoholic steatohepatitis (NASH), cancer, cardiovascular disease (hypertriglyceridemia), and skin disorders.

An exemplary DNL Index was generated DNL=a(16:0) + b(16:1n7) - c(18:2n6),; where the data (i.e. values for the measured levels of the exemplary biomarkers, 16:0, 16:1n7, 18:2n6) was scaled mole %. The scaling and coefficients vary in the final form of the equation to yield close to equal weight for each component in the equation. For example, if the biomarker 16:0 is present as 30% of fatty acids and the biomarker 16:1n7 is present as 1% of fatty acids, the coefficient for 16:1n7 should be approximately 30 to equate the contribution of each fatty acid component of the equation.

The biomarkers were measured using a standard fatty acid analysis platform (GC-FID or GC-MS). The platform produces data on each component of the DNL Index in a single run. Alternatively, an LC-MS platform can be used to identify the species of lipids that best reflect DNL and a biomarker-specific/optimized LC-MS assay can be developed.

Initial estimates of values for scaling the equation were derived. The average concentration (expressed in mole%) of each major DNL fatty acid biomarker in human serum (mean +/- SD) is as follows:

16:0: 21.6 +/- 2.4
16:1n7: 2.1 +/- 1.1
18:2n6 31.9 +/- 4.6

Scaling coefficients can be as simple as dividing each fatty acid component by its mean concentration (or multiplying by the inverse of its concentration). Thus, an example of a DNL Index (based on the average biomarker concentrations measured and reported above) is: DNL Index Score = 0.0463(mole% 16:0) + 0.476(mole% 16:1n7) - 0.0313(mole% 18:2n6). Scaling coefficients may be calculated for any fatty acid including, for example, 16:1n10, 18:1n10, 18:1n12, squalene, 16:0, 16:1n7, 14:0, 14:1n5, 18:2n6, 20:4n6, and 22:6n3 in any sample type.

The methods described in this example may be used to generate a DNL Index for any sample type and using any combination of DNL fatty acid biomarkers.

For Examples 2-3, the following DNL Index was used: DNL Index Score= 0.0463(mole% 16:0) + 0.476(mole% 16:1n7) - 0.0313(mole% 18:2n6).

## Example 2: Effects of Inhibition of De Novo Lipogenesis on DNL Index Score

Two experiments with cells treated with chemical inhibitors of acetyl:coA carboxylase (ACC) were performed. ACC is an enzyme that catalyzes the reaction that produces malonyl-CoA, which is the required substrate for de novo lipogenesis. By inhibiting ACC, malonyl-CoA is not produced which, in turn, inhibits de novo lipogenesis.

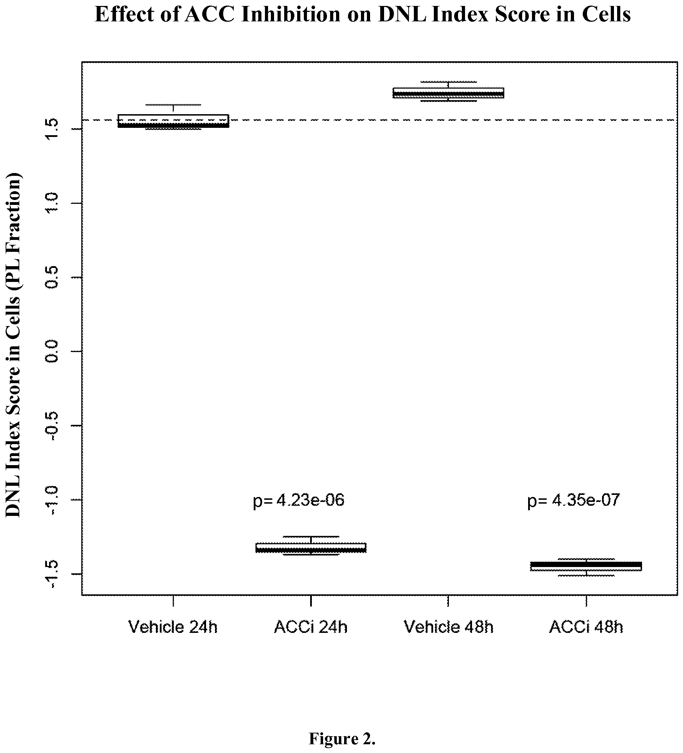

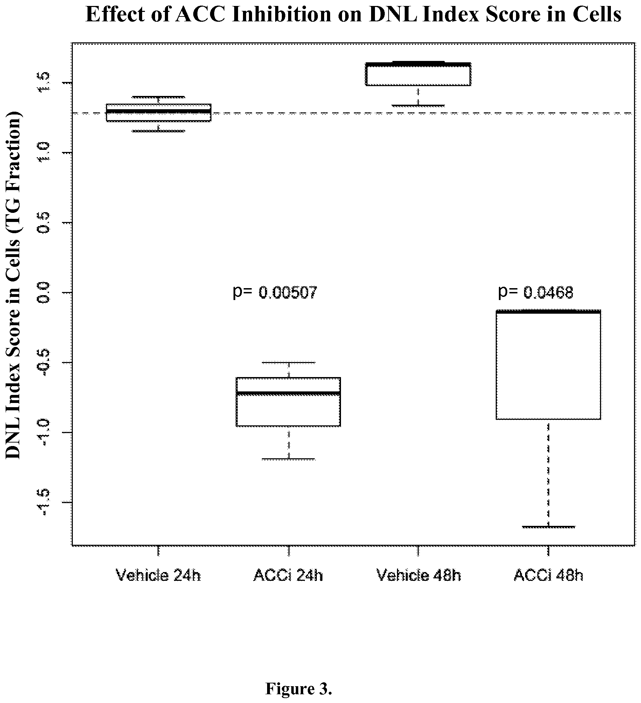

In the first experiment, cells in culture were treated with the inhibitor or the vehicle alone for 24h or 48h. Following treatment, cells were collected and the biomarkers were measured. The levels of the biomarkers were used in the DNL Index to produce the DNL Index Score. The DNL Index Score was significantly lower in the inhibitor treated cells than in the vehicle only control cells, indicating that the de novo lipogenesis was decreased in ACC-inhibitor treated cells. The DNL Index Score was calculated using two lipid fractions, the PL fraction and TG fraction, and similar results were obtained. The data are graphically presented in Figure 2 (PL Fraction) and Figure 3 (TG Fraction).

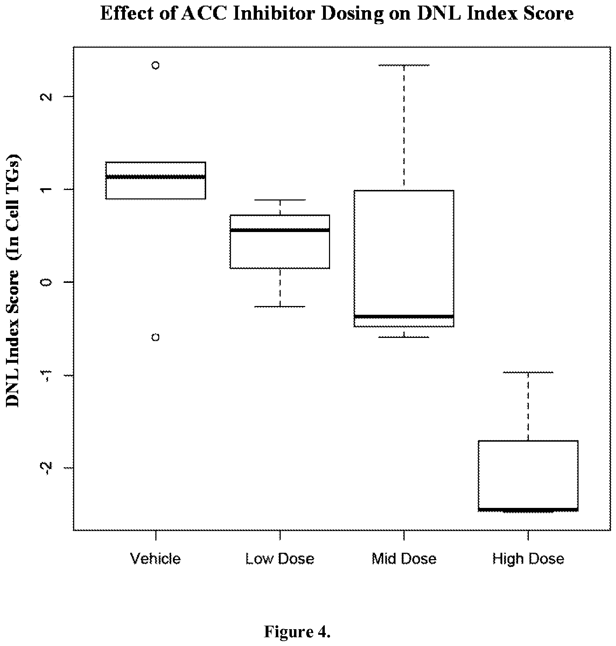

In the second experiment, sebocyte cells in culture were treated with one of three doses (Low, Medium, High) of an ACC inhibitor or a Vehicle only control. After treatment, the cells were collected and the biomarkers were measured. The biomarker levels were used in the DNL Index to produce the DNL Index Score. Cells treated with the inhibitor showed a dose dependent and significant reduction in the DNL Index Score compared to the DNL Index Score obtained with the Vehicle only cells. The data is graphically presented in Figure 4.

## Example 3: Application of DNL Index to Subjects with Liver Disorders

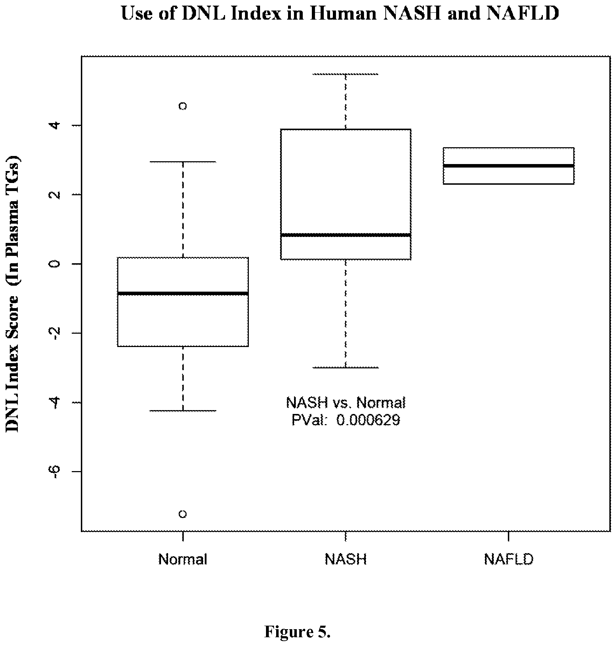

Non-alcoholic Steatohepatitis (NASH) is a disease that starts with the accumulation of fat (thought to be in part DNL fat) in the liver and progresses to inflammation and fibrosis. Blood plasma was collected from a total of 60 subjects with biopsy-confirmed staging of the disease (19 subjects with NASH, 2 subjects with NAFLD, and 39 Normal subjects). The biomarkers 16:0, 16:1n7 and 18:2n6 were measured, and the measurements were used in the DNL Index to generate a DNL Index Score for each subject. There was a clear elevation of the DNL Index Scores in NASH and NAFLD patients relative to their normal controls. The results are graphically illustrated in Figure 5.

## Example 4: Development and Application of DNL Index in Insulin Resistant and Diabetic Subjects

In another example, DNL Index models were generated and applied to a cohort comprised of 749 subjects, 102 of whom progressed to diabetes within 5 years. The cohort was comprised of 55% females, 45% males; 42% white, 34% nonwhite Hispanics, 24% African American; mean age 55. Measures of insulin sensitivity ($S_I$) and acute insulin response (AIR) were obtained from all participants at baseline by frequently sampled intravenous glucose tolerance test (FSIGTT) with minimal model analysis. Of the 102 participants who developed diabetes, the subjects were older with a higher BMI and their baseline values of fasting blood glucose, $S_I$ and AIR were significantly different from individuals who did not develop diabetes.

Fatty acids were quantified as described in Example 1 in serum collected at baseline from the 749 subjects. Fatty acid concentrations differed across the race/ethnic groups; the data are presented in Table 1. The fatty acid biomarkers used as variables in the exemplified DNL Index are indicated in bold font.

**Table 1: Mean concentration of DNL biomarkers by ethnicity**

| Marker | Common name | Mean and Standard Deviation (Mole %) | | | |
|---|---|---|---|---|---|
| | | All | African American | Hispanic | Caucasian |
| **18:2n6** | **Linoleic acid** | 29.84±4.26 | 30.36±3.8$^{\Delta\Sigma}$ | 29.35±4.3$^{\delta}$ | 29.94±4.4$^{\delta}$ |
| **16:0** | **Palmitic acid** | 21.54±2.04 | 21.02±1.7$^{\Delta\Sigma}$ | 21.88±2.0$^{\delta}$ | 21.59±2.1$^{\delta}$ |
| 14:0 | Myristic acid | 0.98±0.38 | 0.82±0.42$^{\Delta\Sigma}$ | 1.04±0.38$^{\delta}$ | 1.03±0.34$^{\delta}$ |
| 14:1n5 | Myristoleic acid | 0.07±0.05 | 0.05±0.05$^{\Delta\Sigma}$ | 0.08±0.04$^{\delta}$ | 0.08±0.04$^{\delta}$ |
| **16:1n7** | **Palmitoleic acid** | 2.08±0.92 | 1.60±0.67$^{\Delta\Sigma}$ | 2.38±0.89$^{\delta}$ | 2.17±0.94$^{\delta}$ |
| pairwise comparisons (t - tests) with Bonferroni correction at p <5.4 x 10$^{-4}$; $\delta$, significantly different from African American; $\Delta$, significantly different from Hispanic; $\Sigma$, significantly different from Caucasian | | | | | |

Prior to analysis, mole% values for the metabolites were normalized to mean 0 and variance 1. Si and AIR were also transformed as AIR=sign(AIR)*sqrt(abs(AIR)); and Si= loge (Si+1). The DNL Index was calculated as DNL = 0.0464 (16:0) + 0.489 (16:1n7) - 0.0335 (18:2n6).

Palmitic acid (16:0) and palmitoleic acid (16:1n7) were strongly inversely associated with insulin sensitivity ($\beta_0$ standardized coefficients of -0.39 and -0.25, p<0.001) and were significant positive predictors of incident diabetes (RR 1.64 and 1.77, p <0.0001); linoleic acid (18:2n6) was strongly positively associated with insulin sensitivity ($\beta_0$ standardized coefficient of 0.38, p<0.001) and was a significant inverse predictor of diabetes (RR 0.61, p<0.001). The data is presented in Table 2. Fatty acid biomarkers used as variables in the exemplified DNL Index are indicated in bold font.

**Table 2: DNL biomarkers univariate associations with insulin sensitivity and diabetes**

| Marker | Full name | Standarized $\beta_0$ coefficients for $S_i$, (p-value) | RR for diabetes per standard dev. of metabolite (95%CI) | p - value for RR |
|---|---|---|---|---|
| **18:2n6** | **Linoleic acid** | 0.38 (<0.0001)$^\delta$ | 0.61 (0.52 - 0.79) | <0.0001$^\delta$ |
| 14:0 | Myristic acid | -0.23 (0.002) | 1.34(1.10 - 1.66) | 0.004 |
| **16:0** | **Palmitic acid** | -0.39 (<0.0001)$^\delta$ | 1.64 (1.34 - 2.02) | <0.0001$^\delta$ |
| 14:1n5 | Myristoleic acid | -0.11 (0.12) | 1.31 (1.07 - 1.62) | 0.009 |
| **16:1n7** | **Palmitoleic acid** | -0.25 (0.001)$^\delta$ | 1.77 (1.42 - 2.20) | <0.0001$^\delta$ |
| RR, relative risk; CI, confidence interval; $S_i$, insulin sensitivity Concentrations are expressed as a mole percent of total fatty acids measured<br>$S_i$ insulin sensitivity<br>$^\delta$p <0.0014 is considered significant based on multiple comparison adjustment by Bonferroni Method | | | | |

The DNL biomarkers were used in the DNL Index. The association of the DNL Index was evaluated by logistic regression. Two models were evaluated, one for the unadjusted DNL Index (DNL Index Score =0.0464 (16:0) + 0.489 (16:10n7) - 0.0335 (18:2n6)), and one for the DNL index adjusted for Si and AIR. The unadjusted DNL Index was a significant predictor of incident diabetes (Table 3; OR:1.5982, P-Value: <0.001). The DNL Index was still significant after adjustment for Si and AIR, with an odds ratio of 1.3987 (p-value: 0.0054). The data indicate that the DNL Index is a predictor of incident diabetes that is independent of insulin sensitivity (Si) and beta-cell function (AIR).

**Table 3: Association of DNL Index with Incident Diabetes (Odds Ratios per 1 Standard Deviation).**

| | Odds Ratio (per 1SD) | P-Value |
|---|---|---|
| Unadjusted DNL Index | 1.5982 | <0.001 |
| Adjusted for Si and AIR | 1.3987 | 0.0054 |

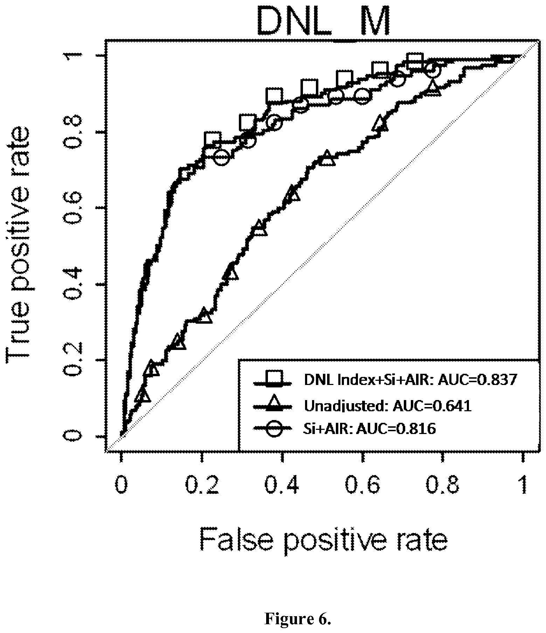

The Area Under the Curve (AUC) was evaluated for the models for prediction of diabetes. The graphic illustration of the Receiver-operator characteristic curve (ROC) is shown in Figure 6. The AUC for model 1, which included only AIR and $S_i$, was 0.816. The AUC for model 2, which was the DNL Index adjusted for Si and AIR was 0.837. Thus, inclusion of the of DNL Index in the mathematical model provided further discrimination of the risk of diabetes beyond the well-known pathogenic basis of diabetes (insulin resistance, beta cell function and obesity), as shown by a significant increase in the ROC between models 1 and 2.

Thus, the markers of de novo lipogenesis (18:2n6, 14:0, 16:0, 14:1n5, and 16:1n7) were associated with incident diabetes, independent of the well-recognized pathway of insulin resistance and impaired beta cell response. The fatty acid biomarkers were cross-sectional correlates of $S_I$ and predictors of diabetes. The results of applying the DNL Index comprised of said biomarkers to said subjects showed that the DNL Index is a) correlated with insulin resistance, and b) predictive of future diabetes in a way that is INDEPENDENT of insulin sensitivity and beta-cell function. The DNL Index represents a new physiological attribute of diabetic processes that should be measured to assess risk of developing diabetes and to monitor changes therein.

**Example 5. Development and Application of Sebum DNL Index.**

In another example, the effects of a lipogenesis inhibitor to inhibit the production / secretion of sebum in skin was assessed using Sebum DNL Index models. The cohort was comprised of 20 subjects enrolled in a clinical study of an inhibitor of lipogenesis as a treatment for reducing sebum secretion. Ten subjects received a placebo and ten subjects received the treatment. A sebum DNL Index Score was calculated for the samples collected at baseline and the samples collected post-treatment. Two sebum DNL Index models were generated and evaluated:

$$\text{Sebum DNL Index 1 (SDI1): DNL Score} = 0.0398(16{:}0) + 0.0455(16{:}1n10) - 1.398(18{:}2n6)$$

$$\text{Sebum DNL Index 2 (SDI2): DNL Score} = 0.0398(16{:}0) + 0.0455(16{:}1n10) - 0.0113(\text{squalene}).$$

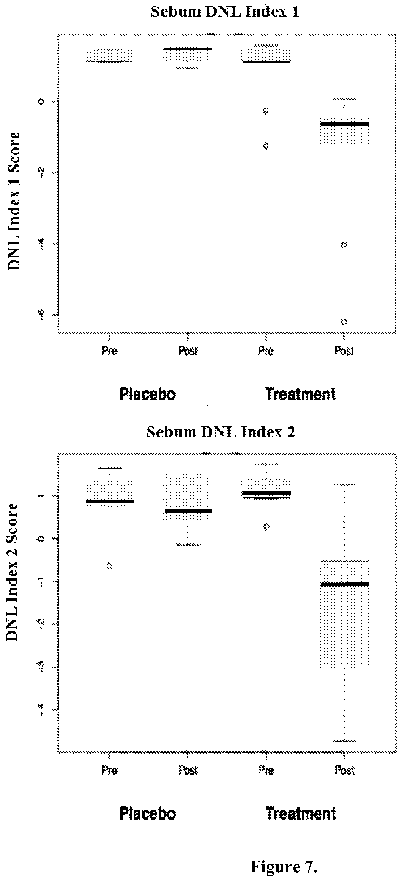

Samples were collected from participants at baseline and post-treatment using Sebutape which was applied to the forehead of participants for 30 minutes, removed and processed via the same methods as described in the General Methods. The measured values obtained for the biomarker variables indicated in each of the Sebum DNL Index models were then used to calculate the DNL Index Scores. With both of the exemplary Sebum DNL Index models, a significant reduction ($p < 0.01$) in DNL Score was observed in the treated group but no change was observed in the DNL Index Score of the placebo group ($p = 0.66$ for SDI1, $p = 0.93$ for SDI2), based on a paired Student's t-test comparing baseline with post-treatement time points for each group. For Sebum DNL Index 1 a significant decrease in the Score was measured with treatment ($p < 0.01$), while there was no effect of placebo ($p = 0.66$) on the SDI1 Score. A graphic representation of the results is illustrated in Figure 7. Similarly, a significant reduction in the Score obtained from the Sebum DNL Index 2 was measured in response to treatment ($p < 0.01$), while the SDI 2 Score did not change in the placebo treatment ($p = 0.93$). The graphical illustration of the data is presented in Figure 7. These results obtained with SDI1 and SDI2 are consistent with the effect of the treatment compared to the placebo.

**Example 6. Performance of DNL Indices in a normal human population**

Human plasma samples from 11 patients whose fractional contribution of *de novo* lipogenesis (fDNL) to total plasma lipid composition was known were used in the analysis. The fDNL was determined as described in [M. K. Hellerstein, De novo lipogenesis in humans: metabolic and regulatory aspects. European journal of clinical nutrition 53 Suppl 1, S53-65 (1999)]. Plasma samples for fatty acid analysis were taken at the same time as the fDNL assessment was made. Fatty acid analysis was performed on each sample to determine the concentration of 16:0, 16:1n7, 18:2n6, 14:0, 14:1n5, 18:1n9, 20:4n6 and 22:6n3.

Using the methods described herein, three versions of the DNL index from the fatty acid composition data were calculated:

$$\text{DNL Index 1 Score} = a(16{:}0) + b(16{:}1n7) - c(18{:}2n6)$$

$$\text{DNL Index 2 Score} = a(16{:}0) + b(16{:}1n7) - c(18{:}2n6) + d(14{:}0) + e(14{:}1n5)$$

$$\text{DNL Index 3 Score} = a(16{:}0) + b(16{:}1n7) - c(18{:}2n6) + d(14{:}0) + e(14{:}1n5) - f(20{:}4n6) - g(22{:}6n3)$$

The coefficients used in the DNL Indices equaled the inverse of the average mole% concentration of each fatty acid across the samples from the study cohort: $a = 0.043870068$, $b = 0.576706455$, $c = 0.030067766$, $d = 0.887528921$, $e = 16.64691481$, $f = 0.178183345$, $g = 0.670499855$.

The DNL Index Score for each of the 11 patients was calculated for the 3 exemplary DNL Indices. Shown in Table 4 are the three DNL Index Scores and the fatty acid concentrations of 16:0, 16:1n7, 18:2n6, 14:0, 14:1n5, 20:4n6, and 22:6n3 for each patient.

**Table 4. DNL Index Scores and fatty acid concentrations for human patients**

| Patient | DNL Index Score | | | Fatty acid concentration | | | | | | |
|---------|---------|---------|---------|---------|---------|---------|---------|---------|---------|---------|
| | 1 | 2 | 3 | 16:0 | 16:1n7 | 18:2n6 | 14:0 | 14:1n5 | 20:4n6 | 22:6n3 |
| 1 | 1.163284 | 3.862224 | 0.71306 | 3151.155 | 215.4047 | 3710.788 | 201.0215 | 10.31253 | 1293.801 | 265.3985 |
| 2 | 1.277512 | 3.776299 | 2.143355 | 2827.337 | 283.1162 | 4273.203 | 171.9636 | 9.493363 | 536.2592 | 160.2696 |
| 3 | 0.550546 | 2.011614 | 0.004254 | 2840.067 | 153.6234 | 4764.346 | 103.5225 | 5.629953 | 635.2783 | 211.4846 |
| 4 | 0.484425 | 1.69259 | -0.23944 | 2835.05 | 118.1739 | 4434.603 | 66.09926 | 5.343228 | 740.3281 | 155.3171 |
| 5 | 1.170412 | 2.791465 | 0.500372 | 3250.218 | 251.4568 | 4396.24 | 128.1943 | 6.096235 | 876.1833 | 220.8993 |
| 6 | 1.077354 | 2.289096 | 0.465728 | 3850.886 | 230.5824 | 4741.784 | 112.3875 | 4.77393 | 857.3678 | 174.3625 |
| 7 | 1.045032 | 2.312099 | -0.42727 | 3752.963 | 229.8355 | 4702.852 | 107.658 | 5.606802 | 1128.539 | 309.141 |
| 8 | 1.369161 | 3.290882 | 0.952936 | 3541.402 | 312.1384 | 4488.297 | 166.9642 | 7.996686 | 1280.518 | 170.1218 |
| 9 | 1.057554 | 2.814825 | 0.689578 | 3377.608 | 279.1935 | 5092.886 | 138.3527 | 8.200776 | 831.1023 | 246.8623 |
| 10 | 0.735557 | 2.151951 | 0.176442 | 3393.518 | 209.4039 | 5387.642 | 101.962:3 | 7.015458 | 812.05 | 215.3759 |
| 11 | 1.575848 | 4.139605 | 2.096358 | 4165.409 | 424.7156 | 5359.345 | 179.1593 | 16.49612 | 790.9214 | 305.2253 |

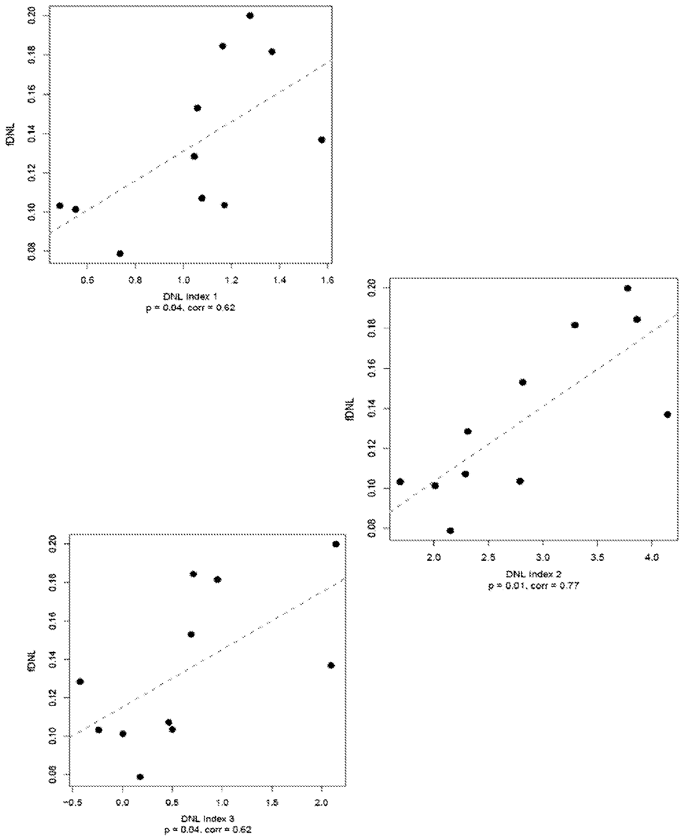

Correlation analysis with each of the three DNL Indices and the fDNL was performed. All three DNL Indices were significantly and positively correlated with fDNL. DNL Index 1 had a correlation coefficient of 0.62 (p = 0.04) with fDNL. DNL Index 2 had a correlation coefficient of 0.77 (p = 0.01) with fDNL. DNL Index 3 had a correlation coefficient of 0.62 (p = 0.04) with fDNL. Figure 8 shows the graphic illustration of the correlation plots for DNL Index 1, DNL Index 2 and DNL Index 3.

**Example 7. Performance of DNL Indices in a patient population with suspected NASH.**

In another example, three DNL Index models were evaluated to determine the association of each Index with a diagnosis of NASH versus not NASH (binary classification) and the stage of steatosis. Diagnosis of NASH and the grade of steatosis were determined by liver biopsy and histology for 213 subjects suspected of having NASH. Fasted serum samples were collected from the subjects on the same day as the biopsies were performed. Fatty acid composition profiling of the serum samples was performed and the association of each of the three example DNL Indices and each of the fatty acids comprising the DNL Indices with the presence of NASH (dichotomous outcome of presence or absence) and with steatosis grade (0, 1, 2, 3) were determined.

Using the three DNL Indices:

$$\text{DNL Index 1 Score} = a(16{:}0) + b(16{:}1n7) - c(18{:}2n6)$$

$$\text{DNL Index 2 Score} = a(16{:}0) + b(16{:}1n7) - c(18{:}2n6) + d(14{:}0) + e(14{:}1n5)$$

$$\text{DNL Index 3 Score} = a(16{:}0) + b(16{:}1n7) - c(18{:}2n6) + d(14{:}0) + e(14{:}1n5) - f(20{:}4n6) - g(22{:}6n3)$$

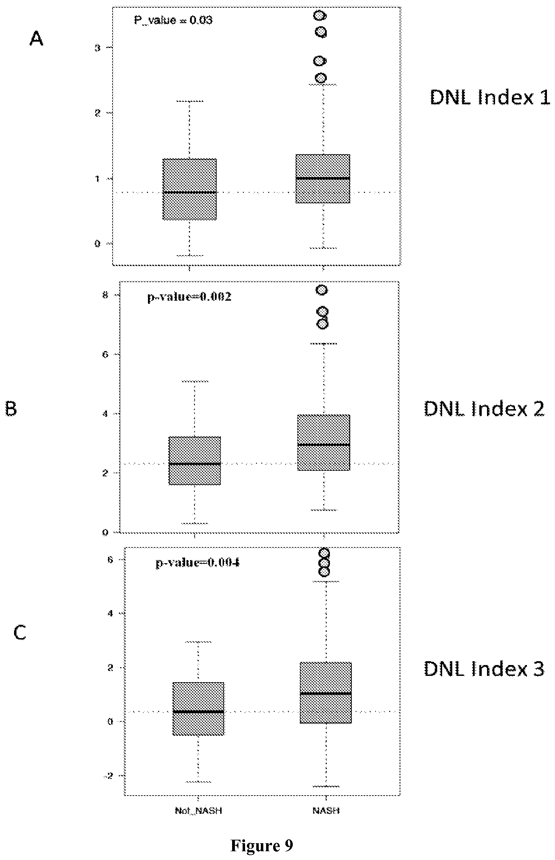

The coefficients used in the DNL Indices equaled the inverse of the average mole% concentration of each fatty acid across the samples from the study cohort: a = 0.043870068, b = 0.576706455, c = 0.030067766, d = 0.887528921, e = 16.64691481, f = 0.178183345, g = 0.670499855.
as described in Example 6, the DNL Index Score for each of the 213 patients was calculated. Each of the three DNL Indices and the individual fatty acid components of the DNL Indices were associated with the NASH diagnosis using logistic regression analysis. The data are presented in Table 5. The results are graphically displayed in Figure 9 which shows boxplots of the distribution of values in NASH and Not-NASH for the Scores from DNL Indices 1, 2 and 3, respectively. Scores from DNL Indices 1, 2 and 3 were all significantly associated with the NASH diagnosis, with p-values of 0.027, 0.002 and 0.004, respectively.

**Table 5. Logistic regression analysis associating DNL Indices with NASH or Not-NASH patient samples**

|  | Mean | | SD | | Logistic |
| --- | --- | --- | --- | --- | --- |
|  | **NotNash** | **Nash** | **NotNash** | **Nash** | **P_value** |
| **DNL Index 1** | 0.830 | 1.054 | 0.582 | 0.639 | 0.027 |
| **DNL Index 2** | 2.450 | 3.153 | 1.128 | 1.458 | 0.002 |
| **DNL Index 3** | 0.400 | 1.154 | 1.255 | 1.693 | 0.004 |
| **FA14:0** | 0.879 | 1.143 | 0.276 | 0.448 | <0.001 |
| **FA14:1n5** | 0.939 | 1.212 | 0.549 | 0.681 | 0.008 |
| **FA16:0** | 0.958 | 1.027 | 0.076 | 0.106 | <0.001 |
| **FA16:1n7** | 1.028 | 1.122 | 0.445 | 0.478 | 0.215 |
| **FA18:1n9** | 1.007 | 1.016 | 0.137 | 0.144 | 0.700 |
| **FA18:2n6** | 1.042 | 0.973 | 0.170 | 0.173 | 0.015 |
| **FA20:4n6** | 1.064 | 1.008 | 0.286 | 0.272 | 0.217 |
| **FA22:6n3** | 1.142 | 1.148 | 0.502 | 0.546 | 0.949 |

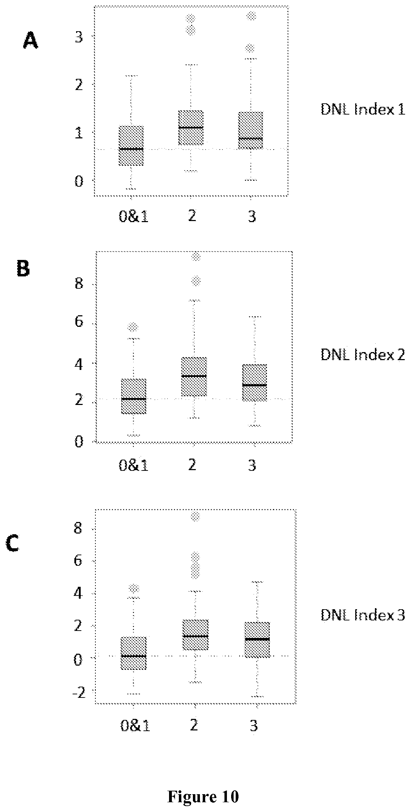

Patients were classified by steatosis grade (Grades 0 & 1, Grade 2, and Grade 3) based on liver biopsy and histology analysis. Each of the three DNL Indices and the individual fatty acid components of the DNL Indices were associated with the steatosis grade using ANOVA analysis. The data are presented in Table 6. The results are graphically displayed in Figure 10 which shows boxplots of the distribution of values in steatosis grades 0 & 1, grade 2, and grade 3 for the Scores from DNL Indices 1, 2 and 3, respectively. Scores from DNL Indices 1, 2 and 3 were all significantly associated with the steatosis grade, with p-values of less than 0.001 for all Indices.

**Table 6. Association of DNL Indices with Steatosis Grade**

|  | Mean | | | SD | | | ANOVA |
|---|---|---|---|---|---|---|---|
|  | G0&1 | G2 | G3 | G0&1 | G2 | G3 | P_value |
| **DNL Index 1** | 0.749 | 1.184 | 1.098 | 0.568 | 0.601 | 0.728 | <0.001 |
| **DNL Index 2** | 2.368 | 3.527 | 3.143 | 1.217 | 1.574 | 1.374 | <0.001 |
| **DNL Index 3** | 0.276 | 1.571 | 1.217 | 1.410 | 1.811 | 1.628 | <0.001 |
| **FA14:0** | 0.897 | 1.257 | 1.110 | 0.334 | 0.499 | 0.354 | <0.001 |
| **FA14:1n5** | 0.918 | 1.373 | 1.185 | 0.585 | 0.809 | 0.549 | <0.001 |
| **FA16:0** | 0.958 | 1.052 | 1.022 | 0.087 | 0.105 | 0.101 | <0.001 |
| **FA16:1n7** | 0.951 | 1.194 | 1.180 | 0.423 | 0.483 | 0.554 | 0.003 |
| **FA18:1n9** | 0.995 | 1.049 | 1.007 | 0.142 | 0.161 | 0.128 | 0.057 |
| **FA18:2n6** | 1.053 | 0.934 | 0.977 | 0.164 | 0.163 | 0.179 | <0.001 |
| **FA20:4n6** | 1.049 | 0.983 | 1.039 | 0.282 | 0.298 | 0.230 | 0.276 |
| **FA22:6n3** | 1.206 | 1.126 | 1.030 | 0.499 | 0.565 | 0.508 | 0.176 |

While the invention has been described in detail and with reference to specific embodiments thereof, it will be apparent to one skilled in the art that various changes and modifications can be made without departing from the scope of the invention.

**Claims**

1. A method of estimating de novo lipogenesis in a subject comprising:

measuring the levels of three or more biomarkers in a biological sample wherein the biomarkers are selected from the group consisting of the total level of myristic acid (14:0), the total level of myristoleic acid (14:1n5), the total level of palmitate (16:0), the total level of palmito leate (16:1n7), the total level of sapienic acid (16:1n10), the total level of squalene, the total level of linoleate (18:2n6), the total level of arachidonic acid (20:4n6), the total level of docosaheaenoic acid (22:6n3), the level of myristic acid (14:0) in one or more lipid class, the level of myristoleic acid (14:1n5) in one or more lipid class, the level of palmitate (16:0) in one or more lipid class, the level of palmitoleate (16:1n7) in one or more lipid class, the level of sapienic acid (16:1n10) in one or more lipid class, the level of squalene in one or more lipid class, the level of linoleate (18:2n6) in one or more lipid class, the level of arachidonic acid (20:4n6) in one or more lipid class, and the level of docosaheaenoic acid (22:6n3) in one or more lipid class in a sample collected from the subject; and using one of the following models to generate a de novo lipogenesis index score to estimate the de novo lipogenesis in the subject:

(i) DNL= a(16:0) + b(16:1n7) - c(18:2n6)
(ii) DNL = (a(16:0) + b(16:1n7)) / c(18:2n6)
(iii) DNL = a(16:0) + b(16:1n7) - c(18:2n6) + d(14:0) + e(14:1n5)
(iv) DNL = a(16:0) + b(16:1n7) - c(18:2n6) + d(14:0) + e(14:1n5)-f(20:4n6) - g(22:6n3)
(v) DNL = a(16:0) + b(16:1n10) - c(18:2n6), or
(vi) DNL = a(16:0) + b(16:1n10) - c(squalene),

wherein a, b, c, d, e, f, and g are constant numbers; (16:0), (16:1n7), (18:2n6), (14:0), (14:1n5), (20:4n6), (22:6n3), (16:1n10), and squalene are measured values of the biomarker analyte; and DNL is the predicted de novo lipogenesis index score.

2. The method of claim 1, wherein the biomarkers comprise the total levels of palmitate (16:0), palmitoleate (16:1n7) and linoleate (18:2n6) in blood.

3. The method of claim 2, wherein the biomarkers further comprise the total levels of myristic acid (14:0), and (14:1n5) in blood.

4. The method of claim 3, wherein the biomarkers further comprise the total levels of arachidonic acid (20:4n6) and docosaheaenoic acid (22:6n3) in blood.

5. The method of claim 1, wherein the biological sample is selected from the group consisting of a sebum sample, a skin sample, an epidermal sample, and a skin cell sample.

6. The method of claim 1, wherein the biomarkers are measured in one or more individual lipid classes selected from the group consisting of triacylglycerides, phosphatidylcholine, and cholesteryl esters.

7. The method of claim 1, wherein the biomarkers are measured after separating the fatty acids from their backbone.

8. The method of claim 1, wherein the biomarkers are measured as an intact complex lipid species.

9. The method of claim 1, wherein the biomarkers are measured in total blood lipids.

10. The method of claim 1, wherein the de novo lipogenesis index score is a sebum de novo lipogenesis index score and is calculated by using model (v) or (vi).

11. The method of claim 1, wherein the de novo lipogenesis index score is used to assess liver health of an individual having liver impairment in the form of non-alcoholic steatohepatitis (NASH), non-alcoholic fatty liver disease (NAFLD), insulin resistance, or diabetes.

12. The method of claim 1, wherein the de novo lipogenesis index score is used for staging hepatic disorders wherein the hepatic disorder is selected from the group consisting non-alcoholic steatohepatitis (NASH) and non-alcoholic fatty liver disease (NAFLD).

13. The method of claim 1, wherein the de novo lipogenesis index score is used to monitor the response to a therapeutic agent on de novo lipogenesis and associated disease conditions.

14. The method of claim 13, wherein the therapeutic agent is a small molecule inhibitor.

15. The method of claim 1, wherein the de novo lipogenesis index score is used to determine the efficacy of a therapeutic agent to inhibit de novo lipogenesis.

**Patentansprüche**

1. Ein Verfahren zum Bewerten von De-novo-Lipogenese bei einem Individuum, das Folgendes beinhaltet:

Messen der Level von drei oder mehr Biomarkern in einer biologischen Probe,
wobei die Biomarker aus der Gruppe ausgewählt sind, die aus Folgendem besteht: dem Gesamtlevel von Myristinsäure (14 : 0), dem Gesamtlevel von Myristoleinsäure (14 : 1n5), dem Gesamtlevel von Palmitat (16 : 0), dem Gesamtlevel von Palmitoleat (16 : 1n7), dem Gesamtlevel von Sapiensäure (16 : 1n10), dem Gesamtlevel von Squalen, dem Gesamtlevel von Linoleat (18 : 2n6), dem Gesamtlevel von Arachidonsäure (20 : 4n6), dem Gesamtlevel von Docosaheaensäure (22 : 6n3), dem Level von Myristinsäure (14 : 0) in einer oder mehreren Lipidklassen, dem Level von Myristoleinsäure (14 : 1n5) in einer oder mehreren Lipidklassen, dem Level von Palmitat (16 : 0) in einer oder mehreren Lipidklassen, dem Level von Palmitoleat (16 : 1n7) in einer oder mehreren Lipidklassen, dem Level von Sapiensäure (16 : 1n10) in einer oder mehreren Lipidklassen, dem Level von Squalen in einer oder mehreren Lipidklassen, dem Level von Linoleat (18:2n6) in einer oder mehreren Lipidklassen, dem Level von Arachidonsäure (20 : 4n6) in einer oder mehreren Lipidklassen und dem Level von Docosaheaensäure (22 : 6n3) in einer oder mehreren Lipidklassen in einer Probe, die von dem Individuum erlangt wurde; und

Verwenden eines der folgenden Modelle zum Erzeugen eines De-novo-Lipogenese-Indexwerts, um die De-novo-Lipogenese bei dem Individuum zu bewerten:

(i) $DNL = a(16:0) + b(16 : 1n7) - c(18 : 2n6)$
(ii) $DNL = (a(16 : 0) + b(16 : 1n7)) / c(18 : 2n6)$
(iii) $DNL = a(16 : 0) + b(16 : 1n7) - c(18 : 2n6) + d(14 : 0) + e(14 : 1n5)$
(iv) $DNL = a(16 : 0) + b(16 : 1n7) - c(18 : 2n6) + d(14 : 0) + e(14 : 1n5) - f(20 : 4n6) - g(22 : 6n3)$
(v) $DNL = a(16 : 0) + b(16 : 1n10) - c(18 : 2n6)$, oder
(vi) $DNL = a(16 : 0) + b(16 : 1n10) - c(Squalen)$,

wobei a, b, c, d, e, f, und g konstante Zahlen sind; (16 : 0), (16 : 1n7), (18 : 2n6), (14 : 0), (14 : 1n5), (20 : 4n6), (22 : 6n3), (16 : 1n10) und Squalen gemessene Werte des Biomarkeranalyts sind; und DNL der vorhergesagte De-novo-Lipogenese-Indexwert ist.

2. Verfahren gemäß Anspruch 1, wobei die Biomarker die Gesamtlevel von Palmitat (16 : 0), Palmitoleat (16 : 1n7) und Linoleat (18 : 2n6) in Blut beinhalten.

3. Verfahren gemäß Anspruch 2, wobei die Biomarker ferner die Gesamtlevel von Myristinsäure (14 : 0) und (14 : 1n5) in Blut beinhalten.

4. Verfahren gemäß Anspruch 3, wobei die Biomarker ferner die Gesamtlevel von Arachidonsäure (20 : 4n6) und Docosaheaensäure (22 : 6n3) in Blut beinhalten.

5. Verfahren gemäß Anspruch 1, wobei die biologische Probe aus der Gruppe ausgewählt ist, die aus einer Talgprobe, einer Hautprobe, einer epidermalen Probe und einer Hautzellenprobe ausgewählt ist.

6. Verfahren gemäß Anspruch 1, wobei die Biomarker in einer oder mehreren einzelnen Lipidklassen, ausgewählt aus der Gruppe, bestehend aus Triacylglyceriden, Phosphatidylcholin und Cholesterylestern, gemessen werden.

7. Verfahren gemäß Anspruch 1, wobei die Biomarker nach dem Trennen der Fettsäuren von ihrer Hauptkette gemessen werden.

8. Verfahren gemäß Anspruch 1, wobei die Biomarker als eine intakte, komplexe Lipidspezies gemessen werden.

9. Verfahren gemäß Anspruch 1, wobei die Biomarker als Gesamtblutlipide gemessen werden.

10. Verfahren gemäß Anspruch 1, wobei der De-novo-Lipogenese-Indexwert ein Talg-De-novo-Lipogenese-Indexwert ist und unter Verwendung von Modell (v) oder (vi) berechnet wird.

11. Verfahren gemäß Anspruch 1, wobei der De-novo-Lipogenese-Indexwert verwendet wird, um die Lebergesundheit eines Individuums, das unter einer Beeinträchtigung der Leber in Form von nicht alkoholischer Steatohepatitis (NASH), nicht alkoholischer Fettlebererkrankung (NAFLD), Insulinresistenz oder Diabetes leidet, zu beurteilen.

12. Verfahren gemäß Anspruch 1, wobei der De-novo-Lipogenese-Indexwert verwendet wird, um hepatische Störungen einzustufen, wobei die hepatische Störung aus der Gruppe ausgewählt ist, die aus nicht alkoholischer Steatohepatitis (NASH) und nicht alkoholischer Fettlebererkrankung (NAFLD) besteht.

13. Verfahren gemäß Anspruch 1, wobei der De-novo-Lipogenese-Indexwert verwendet wird, um die Reaktion auf ein therapeutisches Mittel auf De-novo-Lipogenese und zugehörige Erkrankungszustände zu überwachen.

14. Verfahren gemäß Anspruch 13, wobei das therapeutische Mittel ein niedermolekularer Inhibitor ist.

15. Verfahren gemäß Anspruch 1, wobei der De-novo-Lipogenese-Indexwert verwendet wird, um die Wirksamkeit eines therapeutischen Mittels zur Hemmung von De-novo-Lipogenese zu bestimmen.

**Revendications**

1.  Un procédé d'estimation de lipogenèse *de novo* chez un sujet comprenant :

    la mesure des niveaux de trois biomarqueurs ou plus dans un échantillon biologique dans lequel les biomarqueurs sont sélectionnés dans le groupe constitué du niveau total d'acide myristique (14:0), du niveau total d'acide myristoléique (14:1n5), du niveau total de palmitate (16:0), du niveau total de palmitoléate (16:1n7), du niveau total d'acide sapiénique (16:1n10), du niveau total de squalène, du niveau total de linoléate (18:2n6), du niveau total d'acide arachidonique (20:4n6), du niveau total d'acide docosahexanoïque (22:6n3), du niveau d'acide myristique (14:0) dans une classe de lipides ou plus, du niveau d'acide myristoléique (14:1n5) dans une classe de lipides ou plus, du niveau total de palmitate (16:0) dans une classe de lipides ou plus, du niveau total de palmitoléate (16:1n7) dans une classe de lipides ou plus, du niveau d'acide sapiénique (16:1n10) dans une classe de lipides ou plus, du niveau de squalène dans une classe de lipides ou plus, du niveau de linoléate (18:2n6) dans une classe de lipides ou plus, du niveau d'acide arachidonique (20:4n6) dans une classe de lipides ou plus, du niveau d'acide docosahexanoïque (22:6n3) dans une classe de lipides ou plus dans un échantillon recueilli auprès du sujet ; et
    l'utilisation de l'un des modèles suivants afin de générer un score d'indice de lipogenèse *de novo* afin d'estimer la lipogenèse *de novo* chez le sujet :

    (i) $DNL = a(16:0) + b(16:1n7) - c(18:2n6)$
    (ii) $DNL = (a(16:0) + b(16:1n7)) / c(18:2n6)$
    (iii) $DNL = a(16:0) + b(16:1n7) - c(18:2n6) + d(14:0) + e(14:1n5)$
    (iv) $DNL = a(16:0) + b(16:1n7) - c(18:2n6) + d(14:0) + e(14:1n5) - f(20:4n6) - g(22:6n3)$
    (v) $DNL = a(16:0) + b(16:1n10) - c(18:2n6)$, ou
    (vi) $DNL = a(16:0) + b(16:1n10) - c(squalène)$,

    dans lequel a, b, c, d, e, f, et g sont des nombres constants ; (16:0), (16:1n7), (18:2n6), (14:0), (14:1n5), (20:4n6), (22:6n3), (16:1n10), et squalène sont des valeurs mesurées de l'analyte biomarqueur ; et DNL est le score d'indice de lipogenèse *de novo* prédit.

2.  Le procédé de la revendication 1, dans lequel les biomarqueurs comprennent les niveaux totaux de palmitate (16:0), de palmitoléate (16:1n7) et de linoléate (18:2n6) dans le sang.

3.  Le procédé de la revendication 2, dans lequel les biomarqueurs comprennent en outre les niveaux totaux d'acide myristique (14:0), et de (14:1n5) dans le sang.

4.  Le procédé de la revendication 3, dans lequel les biomarqueurs comprennent en outre les niveaux totaux d'acide arachidonique (20:4n6), et d'acide docosahexanoïque (22:6n3) dans le sang.

5.  Le procédé de la revendication 1, dans lequel l'échantillon biologique est sélectionné dans le groupe constitué d'un échantillon de sébum, d'un échantillon de peau, d'un échantillon épidermique, et d'un échantillon de cellules de peau.

6.  Le procédé de la revendication 1, dans lequel les biomarqueurs sont mesurés dans une ou plusieurs classes de lipides individuelles sélectionnées dans le groupe constitué des triacylglycérides, de la phosphatidylcholine, et des esters de cholestéryle.

7.  Le procédé de la revendication 1, dans lequel les biomarqueurs sont mesurés après la séparation des acides gras de leur squelette.

8.  Le procédé de la revendication 1, dans lequel les biomarqueurs sont mesurés en tant qu'espèce de lipide complexe intacte.

9.  Le procédé de la revendication 1, dans lequel les biomarqueurs sont mesurés dans les lipides totaux du sang.

10. Le procédé de la revendication 1, dans lequel le score d'indice de lipogenèse *de novo* est un score d'indice de lipogenèse *de novo* de sébum et est calculé en utilisant le modèle (v) ou le modèle (vi).

11. Le procédé de la revendication 1, dans lequel le score d'indice de lipogenèse *de novo* est utilisé pour évaluer la santé du foie d'un individu ayant une insuffisance du foie sous forme d'une stéatohépatite non alcoolique (NASH), d'une stéatose hépatique non alcoolique (NAFLD), d'une résistance à l'insuline ou d'un diabète.

12. Le procédé de la revendication 1, dans lequel le score d'indice de lipogenèse *de novo* est utilisé pour l'établissement du stade des troubles hépatiques dans lequel le trouble hépatique est sélectionné dans le groupe constitué de la stéatohépatite non alcoolique (NASH) et de la stéatose hépatique non alcoolique (NAFLD).

13. Le procédé de la revendication 1, dans lequel le score d'indice de lipogenèse *de novo* est utilisé pour surveiller la réponse à un agent thérapeutique sur la lipogenèse *de novo* et les états de maladie associés.

14. Le procédé de la revendication 13, dans lequel l'agent thérapeutique est un inhibiteur à petite molécule.

15. Le procédé de la revendication 1, dans lequel le score d'indice de lipogenèse *de novo* est utilisé pour déterminer l'efficacité d'un agent thérapeutique à inhiber la lipogenèse *de novo.*

**101** Patient Presents to Clinic or MD

**102** Risk Factors and/or Symptoms

No

Yes

**103** Monitor Periodically (Annually)

**104**
- Measure DNL Index (1 or more) biomarker analyte(s)
- Calculate DNL Index Score

**105** DNL Index Score above threshold

No

**106** Monitor Periodically

Yes

**107** Additional Diagnostic Testing and Evaluation to determine appropriate therapeutic treatment

**108** Intervention Needed?

No

Yes

**109** Therapeutic Intervention: Drugs and/or Lifestyle Changes

Monitor Response **110**

Figure 1.

# Effect of ACC Inhibition on DNL Index Score in Cells

Figure 2.

Figure 3.

**Effect of ACC Inhibitor Dosing on DNL Index Score**

Figure 4.

Figure 5.

**Figure 6.**

**Sebum DNL Index 1**

**Sebum DNL Index 2**

**Figure 7.**

Figure 9

31

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090239253 A

### Non-patent literature cited in the description

- **LYONS JONES et al.** *Am J Med Genet,* 2013, vol. 161A, 1860-1865
- **PETER et al.** *J. Clin. Endocrinol. Metab.,* 2011, vol. 96 (7), E1126-E1130
- **FOLCH ; FOLCH, J. ; M. LEES ; G. H. SLOANE-STANLEY et al.** A simple method for the isolation and purification of total lipids from animal tissues. *J. Biol. Chem,* 1957, vol. 226, 497-509
- **PIERARD, G.E. ; PIERARD-FRANCHIMONT, C. ; KLIGMAN, A.M.** Kinetics of sebum excretion evaluated by the Sebutape--Chromameter technique. *Skin pharmacology : the official journal of the Skin Pharmacology Society,* 1993, vol. 6, 38-44
- **AARSLAND A ; WOLFE RR.** Hepatic secretion of VLDL fatty acids during stimulated lipogenesis in men. *J Lipid Res.,* 1998, vol. 39, 1280-1286
- **WU JH ; LEMAITRE RN ; IMAMURA F ; KING IB ; SONG X ; SPIEGELMAN D ; SISCOVICK DS ; MOZAFFARIAN D.** Fatty acids in the de novo lipogenesis pathway and risk of coronary heart disease: the Cardiovascular Health Study. *Am J Clin Nutr.,* 2011, vol. 94, 431-438
- **AARSLAND A ; WOLFE RR.** Hepatic secretion of VLDL fatty acids during stimulated lipogenesis in men. *J Lipid Res.,* 1998, vol. 39, 1280-1286
- **M. K. HELLERSTEIN.** De novo lipogenesis in humans: metabolic and regulatory aspects. *European journal of clinical nutrition,* 1999, vol. 53 (1), S53-65